# EUROPEAN PATENT APPLICATION

(11) **EP 0 884 311 A2**
(43) Date of publication of application: **16.12.1998**
(21) Application number: 98110184.3
(22) Date of filing: 04.06.1998
(51) Int. Cl.: C07D 249/08

(54) **Triazole Derivatives and their production**

(30) Priority: 06.06.1997 JP 149732/97; 09.10.1997 JP 277573/97
(71) Applicant: Takeda Chemical Industries, Ltd., Osaka-shi, Osaka 541-0045 (JP)
(72) Inventor: Itoh, Katsumi, Toyono-gun, Osaka 563-0104 (JP); Kitazaki, Tomoyuki, Kobe, Hyogo 651-1221 (JP); Matsushita, Yoshihiro, Amagasaki, Hyogo 661-0033 (JP); Hosono, Hiroshi, Toyonaka, Osaka 561-0826 (JP); Mitsudera, Hiroyuki, Toyonaka, Osaka 560-0043 (JP)
(74) Representative: von Kreisler, Alek, Dipl.-Chem.

(57) **Abstract**

A compound of formula (I) or a salt thereof is useful as an intermediate for producing antifungal imidazolone or imidazolidinone with advantages from the industrial point of view. 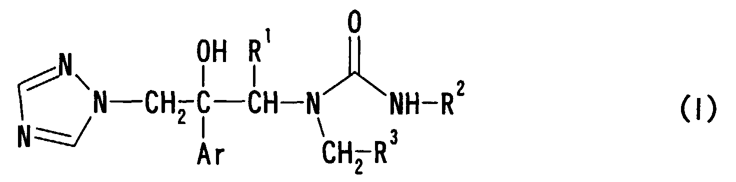 wherein Ar is a phenyl group which may be substituted; R¹ is a hydrogen atom or lower alkyl; R² is an aliphatic or aromatic hydrocarbon group which may be substituted or an aromatic heterocyclic group which may be substituted; R³ is a group of the formula -CH₂Y (wherein Y is a hydroxyl group or a halogen atom) or a formyl group which may be protected.

## Description

The present invention relates to a production technology for antifungal imidazolone or imidazolidinone derivatives and to synthetic intermediates thereof.

A variety of azole derivatives having antifungal activity are presently known. For example, EP-A-567982 (corresponding to Japanese Kokai Tokkyo Koho H6-293740) discloses an azole derivative of the following formula or a salt thereof. 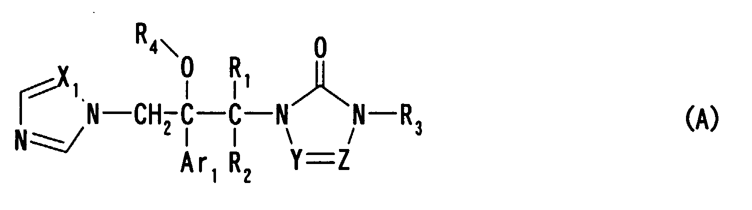 (wherein Ar₁ is a substituted phenyl group; R₁ and R₂ independently are a hydrogen atom or a lower alkyl group, or R₁ and R₂ may combine together to form a lower alkylene group; R₃ is a group bonded through a carbon atom; R₄ is a hydrogen atom or an acyl group; X₁ is a nitrogen atom or a methine group; and Y and Z independently are a nitrogen atom or a methine group which may be optionally be substituted with a lower alkyl group). Japanese Tokkyo Kokai Koho H8-104676 as well as the above patent literature discloses imidazolone derivatives of the following formula as a preferred class of compounds [hereinafter referred to sometimes as compound (B)] and a method for synthesis starting with (R)-lactic acid derivatives. 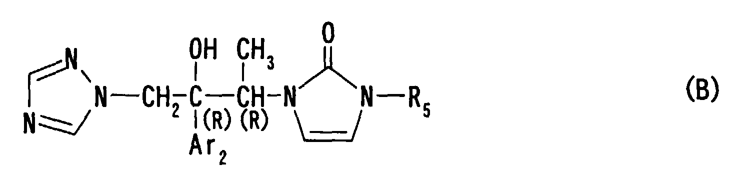 (wherein Ar₂ is halophenyl; R₅ is an aliphatic or aromatic hydrocarbon group which may be substituted or an aromatic heterocyclic group which may be substituted; (R) denotes a configuration).

EP-A-687672 (corresponding to Japanese Kokai Tokkyo Koho H8-253452) describes a process for synthesizing imidazolone derivatives of the above formula (B) using (S)-lactic acid derivatives as the starting material. Moreover, Japanese Kokai Tokkyo Koho H8-104676 mentions imidazolidinone derivatives of the following formula as a preferred class of compounds [hereinafter referred to sometimes as compound (C)] and describes a process for synthesizing them which comprises reducing the above-mentioned compound (B). 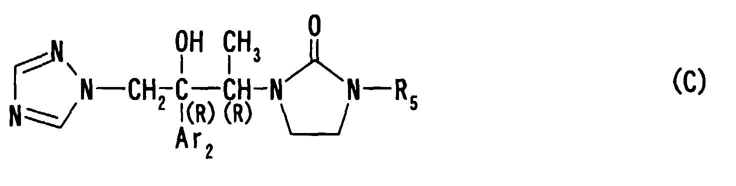 (wherein the respective symbols have the same meanings as defined hereinbefore).

WO 9625410A1 (Japanese Kokai Tokkyo Koho H9-183769) discloses an antifungal azole compound of the following formula, or a salt thereof. 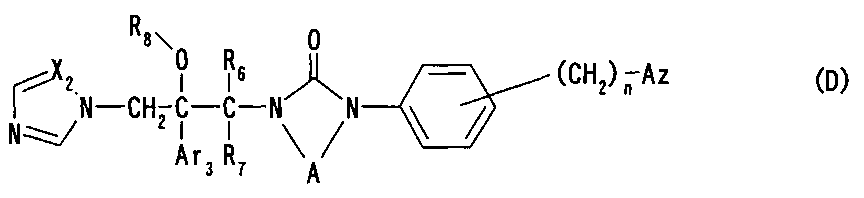 (wherein Ar₃ is an optionally substituted phenyl group; R₆ and R₇, the same or different, are a hydrogen atom or a lower alkyl group, or R₆ and R₇ may be combined together to form a lower alkylene group; R₈ is a hydrogen atom or an acyl group; X₂ is a nitrogen atom or a methine group (-CH=); A is Y=Z (Y and Z, the same or different, are a nitrogen atom or methine group optionally substituted with a lower alkyl group) or an ethylene group optionally substituted with a lower alkyl group; n is an integer from 0 to 2; and Az is an optionally substituted azolyl group). The same patent literature mentions imidazolone derivatives of the following formula (E) as a preferred class of compounds and describes a process for their production which uses (R)-lactic acid derivatives or (S)-lactic acid derivatives as the starting material. 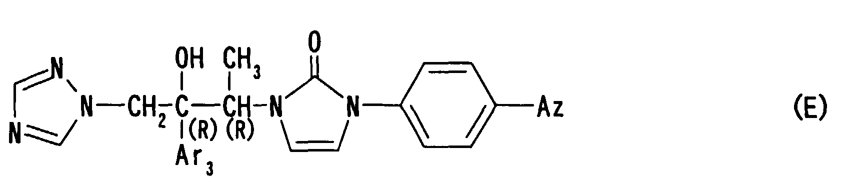 (wherein the respective symbols have the same meanings as defined hereinbefore).

WO 9625410A1 (corresponding to Japanese Kokai Tokkyo Koho H9-183769), referred to above, mentions imidazolidinone derivatives of the following formula (F) as a preferred class of compounds (hereinafter referred to sometimes as compound (F)) and describes a process for synthesizing them which comprises reducing said compound (E). 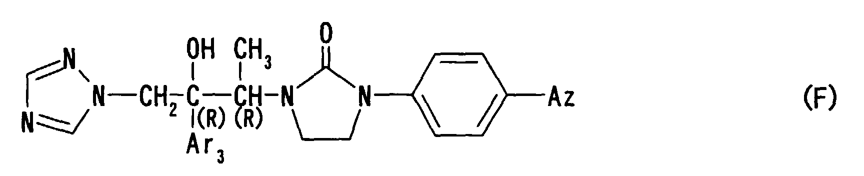 (wherein the respective symbols have the same meanings as defined hereinbefore).

The above patent literature further describes a process for producing imidazolone derivative (B) or (E), which comprises reacting a compound of formula (G) with a compound of formula (H). 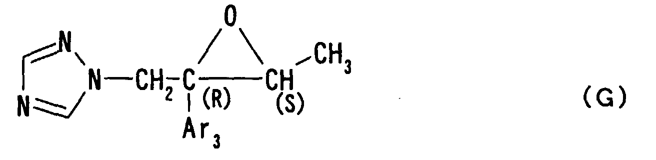 (wherein the respective symbols have the same meanings as defined hereinbefore). 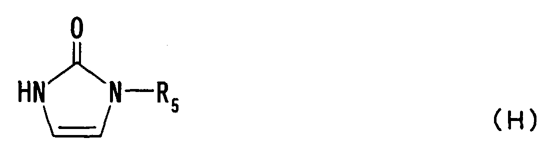 (wherein the respective symbols have the same meanings as defined hereinbefore).

Since the above process for producing compounds (B) and (E) involves reactions under basic conditions, both the starting material and the reaction product undergo partial decomposition so that the yields of compounds (B) and (E) are not as high as desired. Particularly when R⁵ comprises a group labile under basic conditions, the formation of by-products complicates the purification procedure and the product yield is not satisfactory.

Referring to the production technology for imidazolidinone derivatives (C) and (F), the patent literature referred to above describes a process for synthesis of compounds (C) and (F) from compounds (B) and (E), respectively, by catalytic reduction. However, this technology requires a per se expensive metal catalyst (e.g. palladium-on-carbon) and is therefore not satisfactory enough from economic points of view.

The present invention provides a simple and economical method for synthesizing imidazolone and imidazolidinone derivatives (B), (C), (E), and (F), which are useful for therapeutic agents for mycosis, and their synthetic intermediates.

The present invention, therefore, is directed to the following:
(1) A compound of formula: 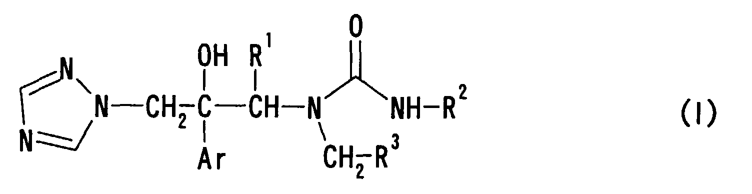 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; R³ is a group of the formula -CH₂Y (wherein Y is a hydroxyl group or a halogen atom) or a formyl group which may be protected, or a salt thereof.
(2) A compound of formula: 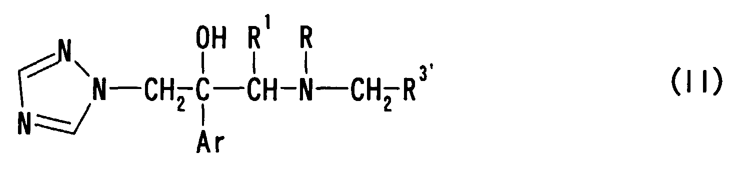 wherein Ar is a phenyl group which may be substituted; R is a hydrogen atom or a benzyl group which may be substituted; R¹ is a hydrogen atom or a lower alkyl group; R^{3'} is a hydroxymethyl group or a protected formyl group, or a salt thereof.
(3) A process for producing a compound of formula: 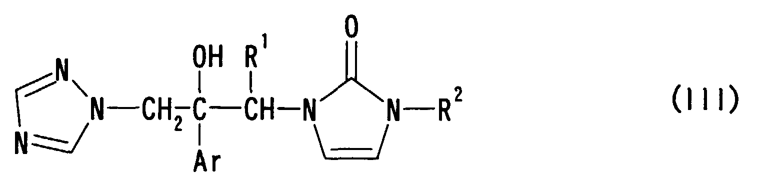 wherein the respective symbols have the same meanings as defined below, or a salt thereof, which comprises subjecting a compound of formula: 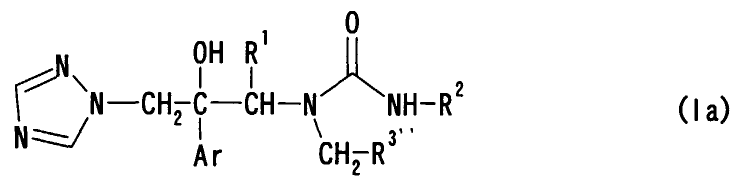 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; R^{3''} is a formyl group which may be protected, or a salt thereof, to a cyclization reaction.
(4) A process for producing a compound of formula: 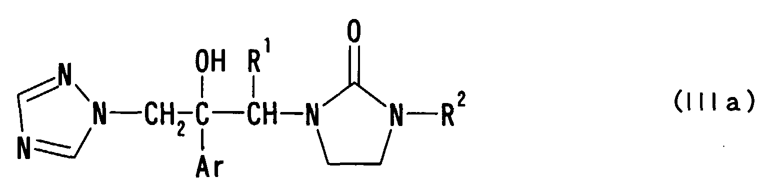 wherein the respective symbols have the same meanings as defined below, or a salt thereof, which comprises subjecting a compound of formula: 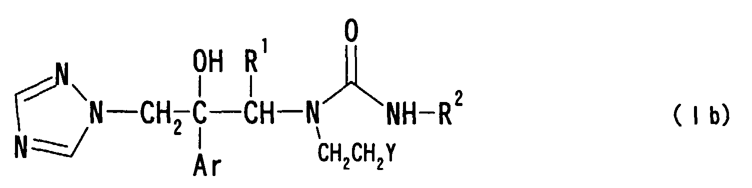 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; Y is a hydroxyl group or a halogen atom, or a salt thereof, to a cyclization reaction.
(5) A process for producing a compound of formula: 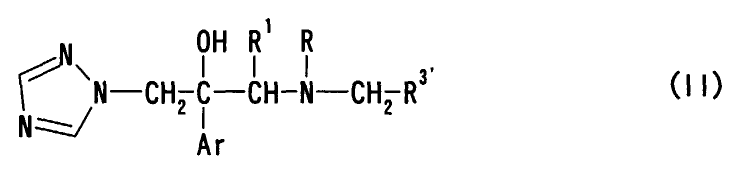 wherein the respective symbols have the same meanings as defined below, or a salt thereof, which comprises reacting a compound of formula: 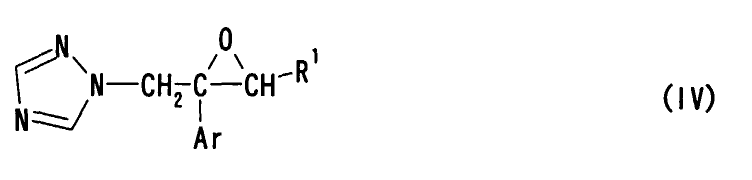 wherein Ar is a phenyl group which may be substituted; R¹ is a hydrogen atom or a lower alkyl group, or a salt thereof, with a compound of formula: 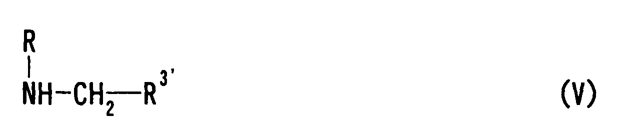 wherein R is a hydrogen atom or a benzyl group which may be substituted, R^{3'} is a hydroxymethyl group or a protected formyl group.
(6) A process for producing a compound of formula: 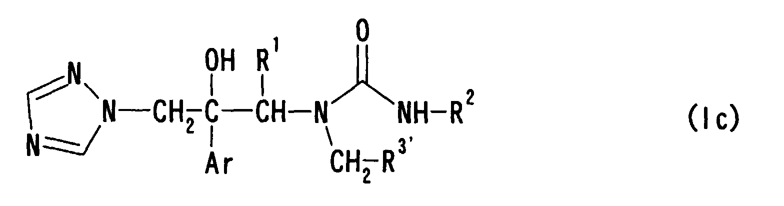 wherein the respective symbols have the same meanings as defined below, or a salt thereof, which comprises reacting a compound of formula: 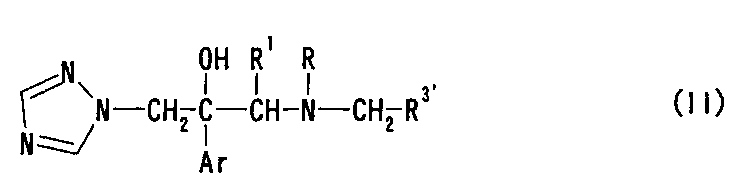 wherein Ar is a phenyl group which may be substituted; R is a hydrogen atom or a benzyl group which may be substituted; R¹ is a hydrogen atom or a lower alkyl group; R^{3'} is a hydroxymethyl group or a protected formyl group, or a salt thereof, with a compound of formula: 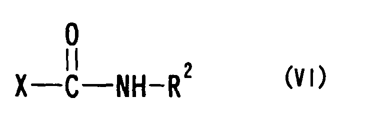 wherein R² is an aliphatic or aromatic hydrocarbon group which may be substituted or an aromatic heterocyclic group which may be substituted; X is a leaving group, or with a compound of formula:

   OCN―R² (VII)

   wherein R² has the same meaning as defined above, if necessary, after the compound of formula (II) or a salt thereof is subjected to a debenzylation reaction.
(7) A process for producing a compound of formula: 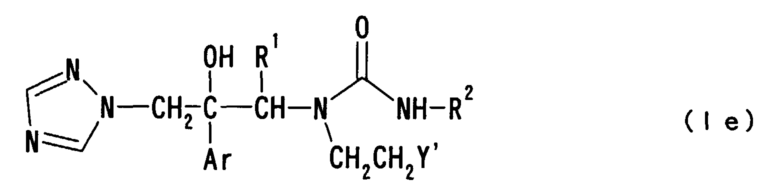 wherein Y' is a halogen atom; the other symbols have the same meanings as below, or a salt thereof, which comprises reacting a compound of formula: 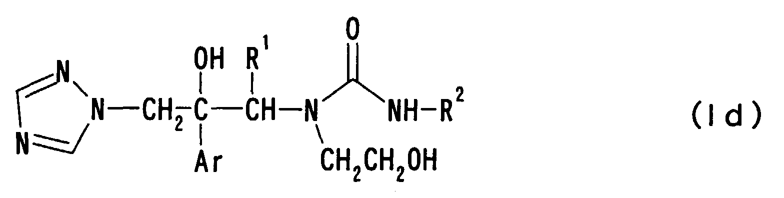 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted, or a salt thereof, with a halogenating agent.
(8) A process for producing a compound of formula: 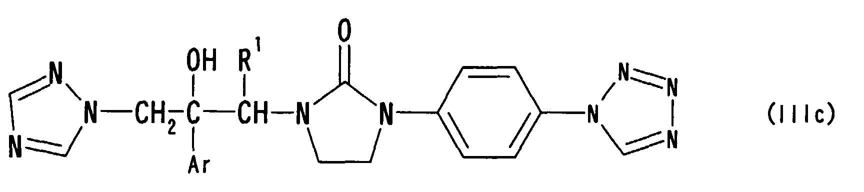 wherein the respective symbols have the same meanings as defined below, or a salt thereof, which comprises reacting a compound of formula: 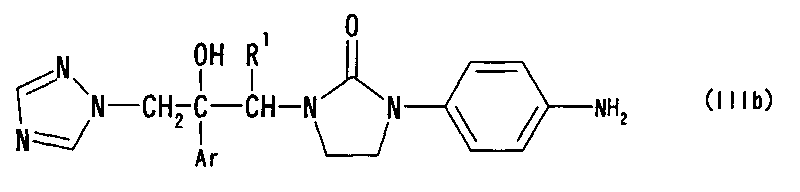 wherein Ar is a phenyl group which may be substituted; R¹ is a hydrogen atom or a lower alkyl group, or a salt thereof, with a lower alkyl ester of orthoformic acid and an alkali metal azide.

Referring to the above formulas (I), (Ia), (Ib), (Ic), (Id), (Ie), (II), (III), (IIIa), (IIIb), (IIIc) and (IV), examples of the substituent group in "a phenyl group which may be substituted" represented by Ar include halogen (e.g. fluorine, chlorine, bromine, iodine, etc.), halo-lower(C₁₋₄) alkyl, halo-lower(C₁₋₄)alkoxy, lower(C₁₋₄)alkylsulfonyl, halo-lower(C₁₋₄)alkylsulfonyl, etc. The substituent group is preferably halogen (e.g. fluorine, chlorine, bromine, iodine, etc.), and more preferably fluorine. The number of substituents is preferably 1 to 3 and more preferably 1 or 2. Examples of Ar include halophenyl, halo-lower(C₁₋₄)alkylphenyl, halo-lower(C₁₋₄)alkoxyphenyl, lower(C₁₋₄)alkylsulfonylphenyl, halo-lower(C₁₋₄)alkylsulfonylphenyl, etc. Examples of halophenyl include phenyl substituted with 1 to 3 halogen atoms selected from fluorine, chlorine, bromine, iodine, etc., such as 2,4-difluorophenyl, 2,4-dichlorophenyl, 4-chlorophenyl, 4-fluorophenyl, 2-chlorophenyl, 2-fluorophenyl, 2-fluoro-4-chlorophenyl, 2-chloro-4-fluorophenyl, 2,4,6-trifluorophenyl, 4-bromophenyl, etc. Particularly preferred examples are phenyl substituted by 1 or 2 fluorine atoms. Examples of halo-lower(C₁₋₄)alkylphenyl include 4-trifluoromethylphenyl, etc.

Examples of halo-lower(C₁₋₄)alkoxyphenyl include 4-trifluoromethoxyphenyl, 4-(1,1,2,2-tetrafluoroethoxy)phenyl, 4-(2,2,2-trifluoroethoxy)phenyl, 4-(2,2,3,3-tetrafluoropropoxy)phenyl, etc. Examples of lower(C₁₋₄⁾ alkylsulfonylphenyl include 4-methanesulfonylphenyl, etc. Examples of halo-lower(C₁₋₄)alkylsulfonylphenyl include 4-(2,2,2-trifluoroethanesulfonyl)phenyl, 4-(2,2,3,3-tetrafluoropropanesulfonyl)phenyl, 4-(2,2,3,3,3-pentafluoropropanesulfonyl)phenyl, etc.

The particularly preferred Examples of Ar include phenyl substituted by 1 or 2 halogen atoms, such as 2,4-difluorophenyl, 2,4-dichlorophenyl, 4-chlorophenyl, 4-fluorophenyl, 2-chlorophenyl, 2-fluorophenyl, 2-fluoro-4-chlorophenyl, 2-chloro-4-fluorophenyl, 4-bromophenyl, etc., and among those groups, 2-fluorophenyl and 2,4-difluorophenyl are most preferred.

R¹ is preferably a lower alkyl group, e.g. a straight-chain or branched C₁₋₄ alkyl group, such as methyl, ethyl, propyl, isopropyl, butyl, isobutyl, sec-butyl and tert-butyl. Particularly preferred is methyl.

Referring to the above formulas (I), (Ia), (Ib), (Ic), (Id), (Ie), (III), (IIIa), (VI) and (VII), examples of aliphatic hydrocarbon group of said "an aliphatic hydrocarbon group which may be substituted" represented by R² include alkyl, cycloalkyl, alkenyl and alkynyl. Examples of alkyl mentioned above include straight-chain or branched alkyl groups of 1 to 12 carbon atoms, such as methyl, ethyl, n-propyl, isopropyl, n-butyl, isobutyl, sec-butyl, tert-butyl, heptyl, octyl, nonyl, decyl, dodecyl, etc. Particularly preferred are lower(C₁₋₄)alkyl groups (e.g. methyl, ethyl, n-propyl, isopropyl, n-butyl, isobutyl, sec-butyl, tert-butyl, etc.). Examples of cycloalkyl mentioned above include cycloalkyl groups of 3 to 8 carbon atoms, such as cyclopropyl, cyclobutyl, cyclopentyl, cyclohexyl, cycloheptyl, cyclooctyl, etc. Particularly preferred are C₃₋₆ cycloalkyl groups (e.g. cyclopropyl, cyclobutyl, cyclopentyl, cyclohexyl, etc.). Examples of the above-mentioned alkenyl include alkenyl groups of 2 to 4 carbon atoms, such as vinyl, propenyl, butenyl, etc. Particularly preferred are C₂₋₃ alkenyl (e.g. vinyl, propenyl, etc.). Examples of the above-mentioned alkynyl include alkynyl groups of 2 to 4 carbon atoms, i.e. ethynyl, propynyl, and butynyl, and among them, C₂₋₃ alkynyl groups (e.g. ethynyl, propynyl, etc.) are preferred.

Examples of the aromatic hydrocarbon group of said "an aromatic hydrocarbon group which may be substituted" represented by R² include groups of 6 to 14 carbon atoms, such as phenyl, naphthyl, biphenyl, anthryl, indenyl, and so forth. Particularly preferred are C₆₋₁₀ aryl (e.g. phenyl, naphthyl, etc.).

Example of the aromatic heterocyclic group of said "aromatic heterocyclic group which may be substituted" represented by R² are groups which are formed by elimination of one hydrogen atom from aromatic heterocycles selected from 5- through 8-membered heterocycles each containing one to several, preferably 1 to 4 hetero atoms such as nitrogen (optionally oxidized), oxygen, sulfur, etc. as well as their fused form and the benzo-fused form. Example of the aromatic heterocyclic group include a variety of aromatic heterocyclic groups such as imidazolyl, triazolyl (1,2,3-triazolyl, 1,2,4-triazolyl), tetrazolyl, pyrazolyl, pyridyl, thiazolyl, isothiazolyl, thiadiazolyl (1,2,4-thiadiazoyl, 1,2,5-thiadiazolyl, 1,3,4-thiadiazolyl), thienyl, furyl, pyrrolyl, pyrazinyl, pyrimidinyl, oxazolyl, isoxazolyl, etc. and fused aromatic heterocyclic groups such as benzimidazolyl, imidazopyrimidinyl, imidazopyridinyl, imidazopyrazinyl, imidazopyridazinyl, benzothiazolyl, quinolyl, isoquinolyl, quinazolinyl, indolyl, and so forth. Preferably, the aromatic heterocyclic group is a 5- or 6-membered aromatic heterocyclic group containing 1 to 3 hetero atoms selected from nitrogen, sulfur, and oxygen (such as imidazolyl, triazolyl, thiazolyl, thiadiazolyl, thienyl, furyl, pyridyl, pyrimidinyl, etc.).

Referring to R², examples of the substituent optionally present on said "an aliphatic or aromatic hydrocarbon group which may be substituted" or on said "aromatic heterocyclic group which may be substituted" include hydroxyl, carboxyl which may be esterified (e.g. carboxy and C₁₋₆ alkoxycarbonyl such as methoxycarbonyl, ethoxycarbonyl, propoxycarbonyl, butoxycarbonyl, etc.), nitro, amino, acylamino (e.g. C₁₋₁₀ alkanoylamino such as acetylamino, propionylamino, butyrylamino, etc.), mono- or di-substituted amino mono- or di-substituted with C₁₋₁₀ alkyl (e.g. methylamino, dimethylamino, diethylamino, dibutylamino, etc.), 5- or 6-membered cyclic amino which may be substituted (e.g. pyrrolidinyl, morpholino, piperidino, pyrazolidinyl, perhydroazepinyl, piperazinyl, 4-benzylpiperazinyl, 4-acetylpiperazinyl, 4-(4-trifluoromethoxyphenyl)-1-piperazinyl, 4-[4-(1,1,2,2-tetrafluoroethoxy)phenyl]-1-piperazinyl, 4-[4-(2,2,3,3-tetrafluoropropoxy)phenyl]-1-piperazinyl, 4-[4-(2,2,2-trifluoroethoxy)phenyl]-1-piperazinyl, 4-[4-(2,2,3,3,3-pentafluoropropoxy)phenyl]-1-piperazinyl, 4-(4-trifluoromethylphenyl)-1-piperazinyl. etc.), C₁₋₆alkoxy (e.g. methoxy, ethoxy, propoxy, butoxy, etc.), halogen (e.g. fluorine, chlorine, bromine, etc.), C₁₋₆ alkyl (e.g. methyl, ethyl, propyl, butyl, etc.), C₃₋₆cycloalkyl (e.g. cyclopropyl, cyclopentyl, etc.), C₆₋₁₄ aryl (e.g. phenyl, naphthyl, indenyl, etc.), halo-C₁₋₆ alkyl (e.g. trifluoromethyl, dichloromethyl, trifluoroethyl, etc.), halo-C₁₋₆ alkoxy (e.g. trifluoromethoxy, 1,1,2,2-tetrafluoroethoxy, 2,2,2-trifluoroethoxy, 2,2,3,3-tetrafluoropropoxy, 2,2,3,3,3-pentafluoropropoxy, 2,2,3,3,4,4,5,5-octafluoropentoxy, 2-fluoroethoxy, etc.), oxo, thioxo, mercapto, C₁₋₆ alkylthio (e.g. methylthio, ethylthio, propylthio, butylthio, etc.), C₁₋₆ alkylsulfonyl (e.g. methanesulfonyl, ethanesulfonyl, butanesulfonyl, etc.), C₁₋₁₀alkanoyl (e.g. acetyl, formyl, propionyl, butyryl, etc.), 5- or 6-membered aromatic heterocyclic groups (e.g. pyrrolyl, pyrazolyl, imidazolyl, 1,2,3-triazolyl, 1,2,4-triazolyl, tetrazolyl, thiazolyl, isothiazolyl, oxazolyl, isoxazolyl, furazanyl, 1,3,4-thiadiazolyl, 1,2,3-thiadiazolyl, 1,2,5-thiadiazolyl, 1,2,4-thiadiazolyl, thienyl, furyl, pyridyl, pyrimidinyl, pyridazinyl, etc.), and fused aromatic heterocyclic groups (e.g. benzimidazolyl, imidazopyrimidinyl, imidazopyridinyl, imidazopyrazinyl, imidazopyridazinyl, benzothiazolyl, quinolyl, isoquinolyl, quinazolyl, indolyl, etc.). Among those substituents, halo-C₁₋₆ alkoxy groups and 5-membered aromatic heterocyclic groups are preferred. Particularly preferred are 1,1,2,2-tetrafluoroethoxy, 2,2,3,3-tetrafluoropropoxy, pyrazolyl (e.g. 1H-pyrazol-1-yl), imidazolyl (e.g. 1H-imidazol-1-yl), 1,2,3-triazolyl (e.g. 1H-1,2,3-triazol-1-yl, 2H-1,2,3-triazol-2-yl), 1,2,4-triazolyl (e.g. 1H-1,2,4-triazol-1-yl), and tetrazolyl (e.g. 1H-tetrazol-1-yl, 2H-tetrazol-2-yl). The number of substituents that may be present is preferably 1 to 3 and more preferably 1 or 2.

Preferred examples of "an aliphatic or aromatic hydrocarbon group which may be substituted and an aromatic heterocyclic group which may be substituted" represented by R², are aromatic hydrocarbon groups which may be substituted. More preferable example are substituted phenyl, and particularly preferable examples are phenyl substituted with halo-C₁₋₆ alkoxy [e.g. 4-(1,1,2,2-tetrafluoroethoxy)phenyl, 4-(2,2,3,3-tetrafluoropropoxy)phenyl] and phenyl substituted with 5-membered aromatic heterocycles [i.e. phenyl substituted with pyrazolyl, imidazolyl, 1,2,3-triazolyl, 1,2,4-triazolyl, or tetrazolyl, such as 4-(1H-pyrazol-1-yl)phenyl, 4-(1H-imidazol-1-yl)phenyl, 4-(1H-1,2,3-triazol-1-yl)phenyl, 4-(2H-1,2,3-triazol-2-yl)phenyl, 4-(1H-1,2,4-triazol-1-yl)phenyl, 4-(1H-tetrazol-1-yl)phenyl, and 4-(2H-tetrazol-2-yl)phenyl].

Referring to the above formulas (I), (Ia), (Ic), (II) and (V), a protected formyl group of said "a formyl group which may be protected" represented by R³ and R³'' or "a protected formyl group" represented by R³' means a formyl group protected by any known formyl-protecting group, and the preferred example of a protected formyl group is what is generally called "acetal". Thus, the protected formyl group includes dialkoxymethyl (e.g. dimethoxymethyl, diethoxymethyl, dipropoxymethyl, dibutoxymethyl, etc.), substituted dialkoxymethyl [e.g. bis(2,2,2-trichloroethoxy)methyl, bis(benzyloxy)methyl, bis(2-nitrobenzyloxy)methyl, etc.], dialkanoyloxymethyl (e.g. diacetoxymethyl etc.), optionally substituted 1,3-dioxan-2-yl [e.g. 1,3-dioxan-2-yl, 5-methylene-1,3-dioxan-2-yl, 5,5-dibromo-1,3-dioxan-2-yl, 5-(2-pyridyl)-1,3-dioxan-2-yl, etc.], optionally substituted 1,3-dioxolan-2-yl [e.g. 1,3-dioxolan-2-yl, 4-(3-butenyl)-1,3-dioxolan-2-yl, 4-phenyl-1,3-dioxolan-2-yl, 4-(2-nitrophenyl)-1,3-dioxolan-2-yl, 4,5-bis(methoxymethyl)-1,3-dioxolan-2-yl, benzo-1,3-dioxosol-2-yl, etc.], 1,5-dihydro-3H-2,4-benzodioxepin-3-yl, bis(alkylthio)methyl [e.g. bis(methylthio)methyl, bis(ethylthio)methyl, bis(propylthio)methyl, bis(butylthio)methyl, bis(pentylthio)methyl, etc.], bis(arylthio)methyl [e.g. bis(phenylthio)methyl etc.], bis(aralkylthio)methyl [e.g. bis(benzylthio)methyl etc.], bis(alkanoylthio)methyl [e.g. bis(acetylthio)methyl etc.], 1,3-dithian-2-yl, 1,3-dithiolan-2-yl, 1,5-dihydro-3H-2,4-benzodithiepin-3-yl, 1,3-oxathiolan-2-yl, and so forth. Particularly preferred are groups of the formula: 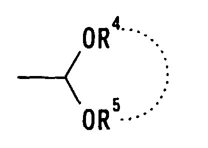 (wherein R⁴ and R⁵ may be the same or different and each is an aliphatic hydrocarbon group or R⁴ and R⁵ combined together represent a lower alkylene group of 2 or 3 carbon atoms.) The aliphatic hydrocarbon group represented by R⁴ and R⁵ includes lower alkyl groups of 1 to 4 carbon atoms (e.g. methyl, ethyl, propyl, butyl, etc.). Among them, methyl and ethyl are preferred, and ethyl is more preferred. The lower alkylene group of 2 or 3 carbon atoms, which is formed with R⁴ and R⁵ combined together, is ethylene or propylene. A protected formyl group of "a formyl group which may be protected" represented by R³ and R³'' or of "a protected formyl group" represented R³' is preferably di-(C₁₋₄)alkoxymethyl, such as dimethoxymethyl, diethoxymethyl, dipropoxymethyl, etc., or a cyclic group such as 1,3-dioxan-2-yl or 1,3-dioxolan-2-yl.

Examples of a benzyl group which may be substituted represented by R include benzyl, p-methoxybenzyl, p-nitrobenzyl, o-nitrobenzyl, p-methylbenzyl, etc., and benzyl is preferred.

A halogen atom represented by Y and Y' in the formulas (I), (Ib) and (Ie) include chlorine, bromine and iodine, and chlorine is preferred.

Referring to formula (VI), a leaving group represented by X is a group which leaves when a compound of formula (VI) reacts with a compound (if necessary, after being subjected to a debenzylation reaction) of formula (II). The leaving group is preferably a group of the formula R⁶-O- (wherein R⁶ is a phenyl group which may be substituted). A phenyl group which may be substituted represented by R⁶ includes a phenyl group which may be substituted by halogen (e.g. chlorine, bromine, etc.), lower alkyl (e.g. methyl, ethyl, etc.) or nitro.

The leaving group X is preferably phenyloxy, chlorophenyloxy, nitrophenyloxy, etc. and is more preferably phenyloxy.

The compound of formula (I) or a salt thereof [hereinafter referred to sometimes as compound (I)] and the compound of formula (II) or a salt thereof [hereinafter referred to sometimes as compound (II)], respectively, contain at least one asymmetric carbon atom and hence may exist as two or more stereoisomers. The respective stereoisomers and a mixture thereof are also involved in the category of compounds of this invention. When R¹ is methyl, optically active compounds in which both the carbon atom linked to the optionally substituted phenyl represented by Ar and the carbon atom linked to the group represented by R¹ have coupled are (R)-configuration, is preferable.

The preferred species of compounds (I) and (II) are those which can be converted to the compounds mentioned as preferred examples of the compound of formula (III) or (IIIa) or its salt [hereinafter referred to briefly as compound (III) or compound (IIIa)], i.e. compounds of formulas (B), (C), (E), and (F). The following is a partial list of particularly preferred species of compound (III).
1-[(1R,2R)-2-(2,4-Difluorophenyl)-2-hydroxy-1-methyl-3-(1H-1,2,4-triazol-1-yl)propyl]-3-[4-(2,2,3,3-tetrafluoropropoxy)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1,1,2,2-tetrafluoroethoxy)phenyl]-2(1H,3H)-imidazolone
1-[(1R,2R)-2-(2-Fluorophenyl)-2-hydroxy-1-methyl-3-(1H-1,2,4-triazol-1-yl)propyl]-3-[4-(2,2,3,3-tetrafluoropropoxy)phenyl]-2(1H,3H)-imidazolone
1-[(1R,2R)-2-(2-Fluorophenyl)-2-hydroxy-1-methyl-3-(1H-1,2,4-triazol-1-yl)propyl]-3-[4-(1,1,2,2-tetrafluoroethoxy)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1H-1,2,3-triazol-1-yl)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1H-tetrazol-1-yl)phenyl]-2(1H,3H)-imidazolone
1-[(1R,2R)-2-(2-Fluorophenyl)-2-hydroxy-1-methyl-3-(1H-1,2,4-triazol-1-yl)propyl]-3-[4-(1H-1,2,3-triazol-1-yl)phenyl]-2(1H,3H)-imidazolone
1-[(1R,2R)-2-(2-Fluorophenyl)-2-hydroxy-1-methyl-3-(1H-1,2,4-triazol-1-yl)propyl]-3-[4-(1H-tetrazol-1-yl)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1H-pyrazol-1-yl)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1H-imidazolyl-1-yl)phenyl]-2(1H,3H)-imidazolone
1-[(1R,2R)-2-(2,4-Difluorophenyl)-2-hydroxy-1-methyl-3-(1H-1,2,4-triazol-1-yl)propyl]-3-[4-(1H-1,2,4-triazol-1-yl)phenyl]-2(1H,3H)-imidazolone

The following is a partial list of particularly preferred species of compound (IIIa).
1-[(1R,2R)-2-(2,4-Difluorophenyl)-2-hydroxy-1-methyl-3-(1H-1,2,4-triazol-1-yl)propyl]-3-[4-(2,2,3,3-tetrafluoropropoxy)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1,1,2,2-tetrafluoroethoxy)phenyl]-2-imidazolidinone
1-[(1R,2R)-2-(2-Fluorophenyl)-2-hydroxy-1-methyl-3-(1H-1,2,4-triazol-1-yl)propyl]-3-[4-(2,2,3,3-tetrafluoropropoxy)phenyl]-2-imidazolidinone
1-[(1R,2R)-2-(2-Fluorophenyl)-2-hydroxy-1-methyl-3-(1H-1,2,4-triazol-1-yl)propyl]-3-[4-(1,1,2,2-tetrafluoroethoxy)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1H-1,2,3-triazol-1-yl)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1H-tetrazol-1-yl)phenyl]-2-imidazolidinone
1-[(1R,2R)-2-(2-Fluorophenyl)-2-hydroxy-1-methyl-3-(1H-1,2,4-triazol-1-yl)propyl]-3-[4-(1H-1,2,3-triazol-1-yl)phenyl]-2-imidazolidinone
1-[(1R,2R)-2-(2-Fluorophenyl)-2-hydroxy-1-methyl-3-(1H-1,2,4-triazol-1-yl)propyl]-3-[4-(1H-tetrazol-1-yl)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1H-pyrazol-1-yl)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1H-imidazol-1-yl)phenyl]-2-imidazolidinone
1-[(1R,2R)-2-(2,4-Difluorophenyl)-2-hydroxy-1-methyl-3-(1H-1,2,4-triazol-1-yl)propyl]-3-[4-(1H-1,2,4-triazol-1-yl)phenyl]-2-imidazolidinone

The compound of formula (III) and the compound of formula (IIIa) can be produced typically in accordance with the following reaction schema. 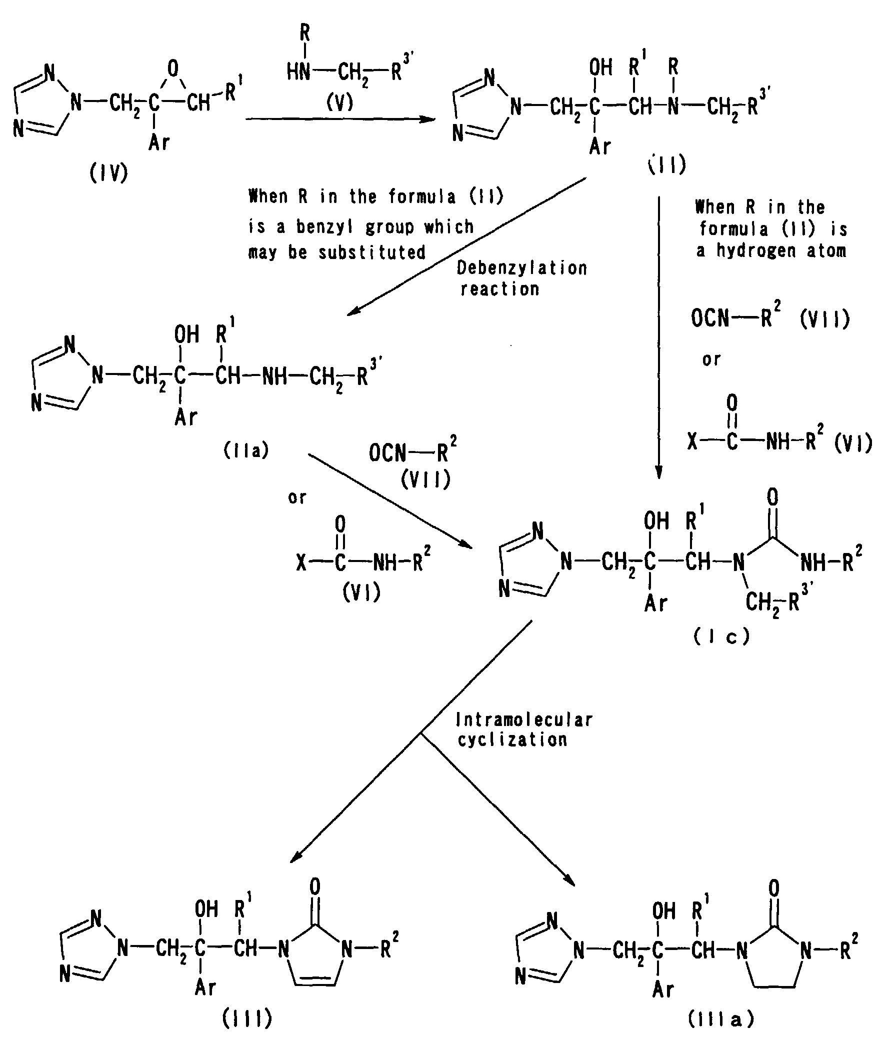 In the above reaction schema, the respective symbols have the same meanings as defined hereinbefore.

Thus, the process comprises ① reacting a compound of formula (IV) or a salt thereof [hereinafter referred to sometimes as compound (IV)] with a compound of formula (V) [hereinafter referred to sometimes as compound (V)] to give compound (II), ② if necessary, after subjecting compound (II) to a debenzylation to give compound (IIa), reacting compound (II) with a compound of formula (VI) [hereinafter referred to sometimes as compound (VI)] or with a compound of formula (VII) [hereinafter referred to sometimes as compound (VII)] to give a compound of formula (Ic) or a salt thereof [hereinafter referred to sometimes as compound (Ic)], ③ if necessary, after deprotecting or halogenating the compound (Ic) to convert to compound (I), subjecting compound (I) to an intramolecular cyclization reaction to give compound (III) or compound (IIIa).

The reaction between compound (IV) and compound (V) is carried out by using about 1 to 100 moles, preferably about 3 to 20 moles, of compound (V) per mole of compound (IV). This reaction is carried out in the presence or the absence of an organometallic compound in a solvent which does not interfere with the reaction or without use of a solvent. Examples of the solvent which does not interfere with the reaction include alcohols such as methanol, ethanol, 1-propanol, 2-propanol, 1-butanol, etc., sulfoxides such as dimethyl sulfoxide etc., ethers such as diethyl ether, tetrahydrofuran, dioxane, etc., nitriles such as acetonitrile etc., aromatic hydrocarbons such as benzene, toluene, xylene, etc., halogenated hydrocarbons such as dichloromethane, chloroform, 1,2-dichloroethane, etc., and amides such as dimethylformamide, acetamide, dimethylacetamide, 1,3-dimethyl-2-imidazolidinone, and 1-methyl-2-pyrrolidone. Among these solvents, alcohols such as methanol, ethanol, 1-propanol, 2-propanol, and 1-butanol are preferred. These solvents can be used each alone or in a suitable combination. The amount of the solvent relative to each part by weight of compound (IV) is usually less than about 100 parts by weight, preferably about 1 to 50 parts by weight, and more preferably about 5 to 20 parts by weight. The reaction temperature is usually about 40 to 200°C and preferably about 70 to 180°C. The reaction time is usually about 1 to 80 hours and preferably about 5 to 50 hours.

Examples of the organometallic compound which may be used in conducting the reaction include titanium alkoxides (e.g. titanium tetraisopropoxide, titanium tetraisobutoxide, etc.) and ytterbium trifluoromethane sulfonate, etc. Particularly preferred are titanium alkoxides. The amount of such an organometallic compound per mole of compound (IV) is about 0.1∼5 equivalents and preferably about 1∼2 equivalents. There is no particular limitation on the mode of addition of said organometallic compound. For example, the organometallic compound may be added after compounds (IV) and (V) have been added to a solvent or added to a solvent before addition of compounds (IV) and (V). As a further alternative, compound (IV), compound (V), and the organometal compound may be sequentially added to a solvent.

The reaction of compound (II) wherein R is a hydrogen atom [hereinafter referred to sometimes as compound (IIa)] with either compound (VI) or compound (VII) is conducted in the presence or the absence of a base in a solvent which does not interfere with the reaction or without use of a solvent. This reaction can be carried out with advantage by using about 0.5 to 5 moles, more preferably about 0.7 to 1.5 moles, of compound (VI) or (VII) per mole of compound (IIa). The base which may be used includes inorganic bases (e.g. potassium carbonate, sodium carbonate, potassium hydroxide, sodium hydroxide, etc.) and organic bases (e.g. triethylamine, pyridine, diisopropylethylamine, etc.). Among them& organic bases such as triethylamine and pyridine are preferred. The amount of the base, if used, is preferably about 0.5∼5 molar equivalents, more preferably about 0.9 to 2 molar equivalents, per mole of compound (IIa).

Examples of the solvent which does not interfere with the reaction of compound (IIa) with compound (VI) or (VII) include sulfoxides such as dimethyl sulfoxide etc., ethers such as diethyl ether, tetrahydrofuran, dioxane, etc., nitriles such as acetonitrile etc., aromatic hydrocarbons such as benzene, toluene, xylene, etc., halogenated hydrocarbons such as dichloromethane, chloroform, 1,2-dichloroethane, etc., esters such as ethyl acetate etc., and amides such as dimethylformamide, acetamide, dimethylacetamide, 1,3-dimethyl-2-imidazolidinone, and 1-methyl-2-pyrrolidone. These solvents can be used each alone or in a suitable combination of two or more species.

Referring to the reaction of compound (IIa) with compound (VI) or (VII), the reaction temperature is usually about -10 to 150°C and preferably about 0 to 120°C. The reaction time is usually about 0.1 to 50 hours and preferably about 0.5 to 30 hours.

When R in compound (II) is a benzyl group which may be substituted, the compound is subjected to a debenzylation reaction to give the compound of formula (II) wherein R is a hydrogen atom, which is represented as compound (IIa). This debenzylation reaction can be easily carried out by any known procedure for elimination of a benzyl group from a benzylated amino group. Such procedures are described in 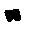 Protective Groups in Organic Synthesis*"*, A. Wiley Interscience Publication.

When R^{3'} in compound (Ic) is protected formyl, the compound can be deprotected to give the compound of formula (I) wherein R³ is formyl. This deprotection reaction can be easily carried out by any known procedure for elimination of a protective group from a formyl group. Such procedures are described in, inter alia, 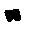 Protective Groups in Organic Synthesis*"*, A. Wiley Interscience Publication.

Among species of compound (Ic), the compound in which R³' is a hydroxymethyl group [compound (Id)] can be optionally halogenated in the hydroxy position using a halogenating agent in a solvent which does not interfere with the reaction to give the compound of formula (I) wherein Y is a halogen atom [compound (Ie)]. This halogenation reaction is carried out by using a halogenating agent to act upon compound [Id] in a solvent which does not interfere with the reaction. Examples of the halogenating agent which can be used for this purpose includes thionyl halides such as thionyl chloride, thionyl bromide, etc. and halogenated phosphorus compounds such as phosphorus trichloride, phosphorus tribromide, phosphorus pentachloride, phosphorus oxychloride, etc. Among those halogenating agents, chlorinating agents such as thionyl chloride and phosphorus oxychloride are preferred. Such a halogenating agent can be used as a diluted solution in an organic solvent (e.g. benzene, toluene, xylene, chloroform, dichloromethane, ethyl acetate, etc.). The amount of the halogenating agent relative to compound (Id) is preferably about 0.5∼5 molar equivalents and more preferably about 0.7 to 3.0 equivalents. The solvent which does not interfere with this reaction includes sulfoxides such as dimethyl sulfoxide etc., ethers such as diethyl ether, tetrahydrofuran, dioxane, etc., ketones such as acetone etc., nitriles such as acetonitrile etc., aliphatic hydrocarbons such as hexane etc., aromatic hydrocarbons such as benzene, toluene, xylene, etc., halogenated hydrocarbons such as dichloromethane, chloroform, 1,2-dichloroethane, etc., esters such as ethyl acetate etc., and amides such as dimethylformamide, acetamide, dimethyl acetamide, 1,3-dimethyl-2-imidazolidinone, and 1-methyl-2-pyrrolidone. Among those solvents, amides such as dimethylformamide, acetamide, dimethylacetamide, 1,3-dimethyl-2-imidazolidinone, 1-methyl-2-pyrrolidone, etc. are preferred. Particularly preferred are dimethylformamide, dimethylacetamide and 1-methyl-2-pyrrolidone. These solvents can be used each alone or in combination. When a mixture of two or more different solvents is used, the mixture preferably consists of one or more amides mentioned as preferred examples as above in a suitable ratio. The reaction temperature suitable for this halogenation reaction is about -10°C to 80°C and preferably about 10° to 60°C. The reaction time is usually about 0.1 to 20 hours and preferably about 0.5 to 8 hours.

The intramolecular cyclization reaction of compound (I) wherein R³ is a formyl group which may be protected [compound (Ia)] to compound (III) is conducted in the presence of an acid in a solvent which does not interfere with the reaction. The acid which can be used for this purpose includes inorganic acids (e.g. hydrochloric acid, sulfuric acid, nitric acid, etc.) and organic acids (e.g. methanesulfonic acid, trifluoromethane sulfonic acid, toluenesulfonic acid, etc.), and inorganic acids such as hydrochloric acid and sulfonic acids are preferred. The acid can be diluted with water and used in the form of an aqueous solution. The amount of the acid per mole of compound (Ia) is preferably about 1∼20 moles and more preferably about 1.0∼10 moles. The solvent which does not interfere with this reaction includes water, alcohols such as methanol, ethanol, propanol, butanol, etc., sulfoxides such as dimethyl sulfoxide etc., nitriles such as acetonitrile etc., ethers such as tetrahydrofuran, dioxane, etc., and ketones such as acetone, etc. Among these solvents, alcohols such as methanol are preferred. Those solvents can be used each alone or in a suitable combination.

This intramolecular cyclization of compound (Ia) can be carried out at a temperature of about 20° to 100°C and preferably about 40° to 80°C. The reaction time is usually about 0.5 to 40 hours and preferably about 1 to 20 hours.

The intramolecular cyclization reaction of compound (I) wherein Y is a halogen atom [compound (Ie)] to compound (IIIa) is conducted in a solvent which does not interfere with the reaction, preferably in the presence of a base. The solvent which does not interfere with this reaction includes sulfoxides such as dimethyl sulfoxide etc., ethers such as diethyl ether, tetrahydrofuran, dioxane, etc., ketones such as acetone etc., nitriles such as acetonitrile etc., aliphatic hydrocarbons such as hexane etc., aromatic hydrocarbons such as benzene, toluene, xylene, etc., halogenated hydrocarbons such as dichloromethane, chloroform, 1,2-dichloroethane, etc., esters such as ethyl acetate etc., and amides such as dimethylformamide, acetamide, dimethylacetamide, 1,3-dimethyl-2-imidazolidinone, and 1-methyl-2-pyrrolidone, etc. These solvents can be used each alone or in a suitable combination. The base which may optionally be used for this reaction includes inorganic bases (e.g. sodium hydride, potassium hydroxide, sodium hydroxide, sodium carbonate, potassium carbonate, etc.) and organic bases (e.g. triethylamine, butylamine, dibutylamine, benzylamine, dimethylaminopyridine, pyrrolidine, N-ethyldiisopropylamine, piperidine, morpholine and so forth). Preferred are organic bases such as triethylamine, dimethylaminopyridine, piperidine, etc. The amount of the base, if used, is preferably about 0.5 to 50 molar equivalents and more preferably about 0.5 to 10 molar equivalents based on compound (Ie). The reaction temperature is usually about -20°C to 100°C and preferably about -10°C to 60°C. The reaction time is usually about 0.1 to 24 hours and preferably about 0.5 to 10 hours.

The intramolecular cyclization reaction of compound (I) wherein Y is a hydroxyl group [compound (Id)] to compound (IIIa) is conducted in the presence of a condensing agent in a solvent which does not interfere with the reaction. The preferred condensing agent is a combination of a halogenating agent and a base or a combination of an azodicarboxylic acid derivative and a phosphine derivative. The solvent which does not interfere with the reaction when the condensing agent is a combination of a halogenating agent and a base includes sulfoxides such as dimethyl sulfoxide etc., ethers such as diethyl ether, tetrahydrofuran, dioxane, etc., ketones such as acetone etc., nitriles such as acetonitrile etc., aliphatic hydrocarbons such as hexane etc., aromatic hydrocarbons such as benzene, toluene, xylene, etc., halogenated hydrocarbons such as dichloromethane, chloroform, 1,2-dichloroethane, etc., esters such as ethyl acetate etc., and amides such as dimethylformamide, acetamide, dimethyl acetamide, 1,3-dimethyl-2-imidazolidinone, 1-methyl-2-pyrrolidone, and so forth. These solvents can be used each alone or in a suitable combination of two or more species. When only one kind of solvent is used, it is preferably selected from among amides such as dimethylformamide, dimethylacetamide, 1-methyl-2-pyrrolidone and so forth. Among such amides, dimethylacetamide and 1-methyl-2-pyrrolidone is particularly preferred. When two or more kinds of solvents are used in combination, it is preferable to use an amide such as dimethylformamide, dimethylacetamide or 1-methyl-2-pyrrolidone in combination with one or more other solvents. The halogenating agent that can be used in this reaction includes thionyl halides such as thionyl chloride, thionyl bromide, etc. and halogenated phosphorus compounds such as phosphorus trichloride, phosphorus tribromide, phosphorus pentachloride, phosphorus oxychloride, etc. Particularly preferred are chlorinating agents such as thionyl chloride and phosphorus oxychloride. The halogenating agent may optionally be used as a diluted solution in an organic solvent (e.g. benzene, toluene, xylene, chloroform, dichloromethane, ethyl acetate, etc.). The amount of the halogenating agent is preferably about 0.5 to 5 molar equivalents and more preferably about 0.7 to 3.0 equivalents relative to compound (Id).

The base which can be used in this reaction includes inorganic bases (e.g. sodium hydride, sodium hydroxide, sodium carbonate, potassium carbonate, etc.) and organic bases (e.g. triethylamine, butylamine, dibutylamine, benzylamine, dimethylaminopyridine, pyrrolidine, N-ethyldiisopropylamine, piperidine, morpholine, etc.). Preferred are organic bases such as triethylamine, dimethylaminopyridine and piperidine. The amount of the base, relative to compound (Id), is preferably about 0.5 to 50 molar equivalents and more preferably about 0.5 to 20 molar equivalents. The order of addition of the halogenating agent and the base is not restricted. Thus, for example, the base may be first added to a solution of compound (Id) and the halogenating agent then added. If desired, this order may be reversed. As a further alternative, both of the base and the halogenating agent may be added simultaneously. It is preferable to add either the base or the halogenating agent to a solution of compound (Id) and then add the other. The reaction temperature is usually about -20°C to 100°C and preferably about -10°C to 60°C. The reaction time is usually about 0.1 to 50 hours and preferably about 1 to 24 hours.

Referring to the case in which the intramolecular cyclization reaction for production of compound (IIIa) from compound (Id) is carried out using an azodicarboxylic acid derivative and a phosphine derivative in combination, the azodicarboxylic acid derivative includes azodicarboxylic esters and amides, such as diethyl azodicarboxylate, dimethyl azodicarboxylate, diisopropyl azodicarboxylate, N,N,N',N'-tetramethylazodicarboxamide, 1,1'-(azodicarbonyl)dipiperidine, N,N,N',N'-tetraisopropylazodicarboxamide, 1,6-dimethyl-1,5,7-hexahydro-1,4,6,7-tetrazocine-2,5-dione, and so forth. The phosphine compound includes triarylphosphines and trialkylphosphines, such as triphenylphosphine, tributylphosphine, trimethylphosphine, and so forth. As reagents alternative to such combinations of azodicarboxylic acid derivatives with phosphine derivatives, there can be mentioned phosphoranes such as cyanomethylenetributyl phosphorane, cyanomethylenetriethyl phosphorane, and so forth. These reagents are used in organic synthetic reactions known generally as the Mitsunobu reaction and are described in 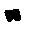 Organic Reactions, Vol. 42*"* and 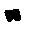 Journal of Synthetic Organic Chemistry, Japan, 55, 631, 1997*"*.

The solvent which does not interfere with this reaction includes ethers such as diethyl ether, tetrahydrofuran, dioxane, etc., ketones such as acetone etc., nitriles such as acetonitrile etc., aliphatic hydrocarbons such as hexane etc., aromatic hydrocarbons such as benzene, toluene, xylene, etc., halogenated hydrocarbons such as dichloromethane, chloroform, 1,2-dichloroethane, etc., and esters such as ethyl acetate and so on. These solvents can be used each alone or in a suitable combination. The amount each of the azodicarboxylic acid derivative and the phosphine derivative relative to compound (Id) is preferably about 0.5 to 5 molar equivalents and more preferably about 1 to 2 molar equivalents. The reaction temperature is about 0° to 50°C and preferably about 10° to 30°C. The reaction time is about 0.1 to 24 hours and preferably about 1 to 5 hours. In conducting this reaction, the order of addition of compound (Id), the azodicarboxylic acid derivative, and the phosphine derivative is not particularly restricted.

Compound (IIIa) wherein R² is 4-aminophenyl [Compound (IIIb)] or a salt thereof can be produced by subjecting the compound (III) wherein R² is 4-nitrophenyl [i.e. compound of the formula: 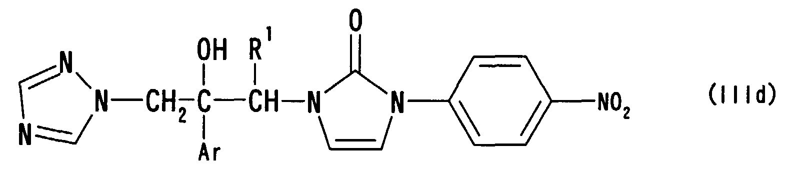 (wherein each symbol has the meaning given above) or a salt thereof] to catalytic reduction in a solvent which does not interfere with the reaction. The solvent which does not interfere with the reaction includes water or an organic solvent such as an alcohol (e.g.:methanol, ethanol, propanol, isopropyl alcohol, butanol etc.), an ester (e.g.: ethyl acetate, etc.), a hydrocarbon (e.g.: benzene, toluene, hexane, xylene etc.), an organic carboxylic acid (e.g.: acetic acid, propionic acid etc.) etc. or a mixture of two or more of these solvents. The reaction can usually be carried out in the presence of a catalyst. Examples of the catalyst are suitable metallic catalyst such as palladium-carbon, platinum-carbon, Raney nickel, etc. The catalytic reduction can be carried out under a pressure ranging normal pressure to about 150kg*/*cm² , at temperatures of room temperature to 100°C.

Furthermore the compound (IIIb) or a salt thereof can be produced by subjecting a compound (IIIa) wherein R² is 4-nitrophenyl [i.e. compound of the formula: 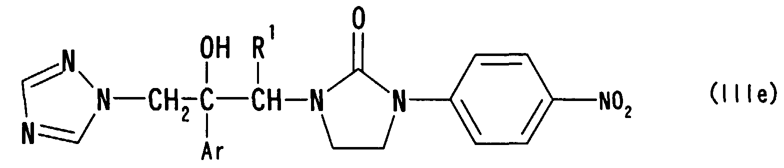 (wherein each symbol has the meaning given above) or a salt thereof] to reduction reaction in a solvent which does not interfere with the reaction. The reduction can usually be carried out in the presence of a reducing agent. Example of the reducing agent include a combination of hydrogen and a metal catalyst (e.g. : palladium-carbon, platinum-carbon, Raney nickel, etc.), a combination of hydrazine and a metal (e.g.: palladium-carbon, ferric chloride etc.), a combination of a metal (e.g.: iron, zinc, tin, aluminum amalgam, Raney-alloy etc.) and an acid (e.g., hydrochloric acid, acetic acid etc.), water or an aqueous alkaline solution (e.g., aqueous solution of sodium hydroxide etc.), etc. The reaction temperature is usually about 0∼100°C, preferably about 10 to 40°C. A solvent which does not interfere with the reaction includes water, an organic solvent such as an alcohol (e.g. : methanol, ethanol, propanol, isopropyl alcohol, butanol, etc.), an ester (e.g. : ethyl acetate etc.), a hydrocarbon (e.g. : benzene, toluene, hexane, xylene etc.), an ether (e.g. : tetrahydrofuran, etc.), a nitrile (e.g. :acetonitrile, etc.), an organic carboxylic acid (e.g.: acetic acid, propionic acid, etc.), etc. These solvents can be selected depend on the reducing agent used, and can be used alone or in combination.

The compound of general formula (IIIa) wherein R² is 4-aminophenyl or a salt thereof [compound (IIIb)] can be reacted with an alkyl orthoformate and a metal aside to give the compound of general formula (IIIa) wherein R² is 4-(1H-tetrazol-yl)phenyl or a salt thereof [compound (IIIc)]. The alkyl orthoformate mentioned above includes lower(C₁₋₄)alkyl esters of orthoformic acid such as methyl orthoformate, ethyl orthoformate, propyl orthoformate, and butyl orthoformate, etc. Among them, C₁₋₂alkyl esters, such as methyl orthoformate, ethyl orthoformate, etc. are preferred. The metal azide includes alkali metal azides such as sodium azide etc. and is preferably sodium azide. This reaction is usually carried out in a solvent. The solvent is preferably an organic acid such as acetic acid, propionic acid, etc., and acetic acid is more preferable. The reaction temperature is generally 0° to 150°C and preferably 20° to 100°C. The reaction time is generally 10 minutes to 24 hours and preferably 1 to 10 hours.

The above-mentioned compounds (III), (IIIa) (IIIb), (IIIc), (IIId) and (IIIe), and their synthetic intermediates (I), (Ia), (Ib), (Ic), (Id), (Ie), and (II) can be respectively isolated and purified from the respective reaction mixtures by per se known procedures such as, for example, extraction, concentration, neutralization, filtration, recrystallization, and chromatography.

Compounds (I), (Ia), (Ib), (Ic), (Id), (Ie), (II), (IIa), (III), (IIIa), (IIIb), (IIIc), (IIId) and (IIIe) can be isolated as salts. The salts include salts with inorganic acids (e.g. hydrochloric acid, hydrobromic acid, sulfuric acid, nitric acid, phosphoric acid, etc.) and salts with organic acids (e.g. acetic acid, tartaric acid, citric acid, fumaric acid, maleic acid, p-toluenesulfonic acid, methanesulfonic acid, etc.).

Salts of compounds (I), (Ia), (Ib), (Ic), (Id), (Ie), (II), (IIa), (III), (IIIa), (IIIb), (IIIc), (IIId) and (IIIe) can be produced by the per se known method, for example by adding the corresponding inorganic or organic acid to compounds of the formulas (I), (Ia), (Ib), (Ic), (Id), (Ie), (II), (IIa), (III), (IIIa), (IIIb), (IIIc), (IIId) and (IIIe), respectively.

Compound (I), (Ia), (Ib), (Ic), (Id), (Ie), (II), (IIa), (III), (IIIa), (IIIb), (IIIc), (IIId) and (IIIe) may each be a hydrate or an anhydride.

While compounds (I) and (II) may exist as at least two stereoisomers, those isomers can be independently prepared. For example, by carrying out the above reaction using one isomer of the starting compound (IV), the corresponding isomer of compound (I) or (II) of the invention can be produced. When the product is a mixture of two or more isomers, it can be separated into the respective isomers by a conventional separation method, for example by using the method of forming a salt with an optically active acid (e.g. camphorsulfonic acid, tartaric acid, etc.), chromatography, fractional recrystallization, and so forth.

The preferred antifungal compounds (III) and (IIIa) which can be produced by the method of this invention are an optically active compounds wherein R¹ is a methyl group, in which both the carbon atom linked to Ar and the carbon linked to R¹ have (R)-configuration, as mentioned hereinbefore. These compounds can be synthesized starting from an optically active oxirane derivative (IV) wherein R¹ is a methyl group, in which the carbon linked to Ar has (R)-configuration and the carbon linked to R¹ has (S)-configuration, through optically active synthetic intermediates of formula (II) and (I) in which both the carbon linked to Ar and the carbon linked to R¹ (methyl) have (R)-configuration.

The starting compound (IV) for use in the present invention can be synthesized by the per se known technology, for example by the processes described in Chemical and Pharmaceutical Bulletin, 41, 1035-1042, 1993 and 43, 432-440, 1995, or any process analogous thereto. The other starting compound (VI) can also be produced by the per se known technology, for example by the processes described in Chemical and Pharmaceutical Bulletin, 44, 314-327, 1996 and WO 9625410A, or any process analogous thereto.

Since the compounds (III) and (IIIa) produced in the present method have low toxicity and strong antifungal activity to the genus Candida [e.g. Candida albicans, Candida utilis, Candida graburata, etc.], genus Histoplasma [e.g. Histoplasma capsulatum, etc.], genus Aspergillus [e.g. Aspergillus niger, Aspergillus fumigatus, etc.], genus Cryptococcus [e.g. Cryptococcus neoformans, etc.], genus Trichophyton [e.g. Trichophyton rubrum, Trichophton mentagrophytes. etc.], genus Microsporum [e.g. Microsporum gypseum, etc.], genus Mallassezia [e.g. Mallassezia furfur, etc.], etc., it can be used for prevention or treatment of fungal infections [e.g. mucosal candidiasis (oral thrush, angular stomatitis, vulvovaginal candidiasis, candida balanoposthitis and urethritis), dermal candidiasis (interdigital candidiasis, intertriginous candidiasis, perianal candidiasis, blastomycosis cutis eczematosa, candida onychia, candida paronychia, auricular candidiasis, cutaneous lesion of candida septicaemia, diffuse superficial candidiasis, candida granuloma, congenital cutaneous candidiasis, candidids), chronic mucocutaneous candidiasis and systemic candidiasis (candidiasis of the respiratory tract, candidiasis of the gastrointestinal tract,candida septicaemia, candida endocarditis, candidiasis of the urinary tract, candidiasis of the eye, candidiasis of the central nervous system, articular and bone candidiasis, candida peritonitis, candidiasis of the liver, intrauterine candidiasis, etc.) due to genus Candida; acute pulmonary histoplasmosis, chronic pulmonary histoplasmosis and disseminated histoplasmosis, etc. due to genus Histoplasma; aspergillosis of the respiratory tract (allergic aspergillosis, bronchial aspergillosis, aspergilloma, pulmonary aspergillosis (acute invasive pulmonary aspergillosis, chronic necrotizing pulmonary aspergillosis), aspergillary empyema), disseminated aspergillosis, central nervous system aspergillosis, aspergillary endocarditis, aspergillary myocarditis, aspergillary pericarditis, aspergillary mycetoma, aspergillary otomycosis, aspergillary onychia, aspergillary paronychia, aspergillary keratitis, aspergillary endophthalmitis, cutaneous aspergillosis and nasal-orbital aspergillosis, etc. due to genus Aspergillus; pulmonary cryptococcosis, central nervous system cryptococcosis, cutaneous and mucocutaneous cryptococcosis, osseous cryptococcosis, cryptococcosis of lymphnodes, disseminated cryptococcosis and cryptococcosis of hematopoetic organs, etc. due to genus Cryptococcus; tinea capitis, favus, kerion celsi, tinea barbae, trichophytia maculovesiculosa, trichophytia eczematosa marginata, tinea imbricata, trichophytia pompholyciformis, tinea unguium, trichophitid and granuloma trychophyticum, etc. due to genus Trichophyton or genus Microsporum; tinea versicolor, etc. due to genus Mallassezia] in the mammals (e.g. human, domestic animal, fowl, etc.), and can also be used for prevention or treatment of atopic dermatitis. Furthermore, two or more compounds among compounds (III) or (IIIa) can be used as an antifungal agent in combination, and one or more compounds among compounds (III) and (IIIa) can be used as an antifungal agent in combination with one or more compounds having antifungal activity other than compounds (III) and (IIIa).
Compounds (III) and (IIIa) can also be used as an agricultural antifungal agent.

The experimental pharmacological efficacy and action, compatible patient background, safety, indications, dosage, route of administration, dosage form and other treatment modalities, etc. and the mode of use as agricultural fungicides of compounds (III) and (IIIa) are as described in the above-mentioned Japanese Kokai Tokkyo Koho H6-293740, Kokai Tokkyo Koho H8-104676, and WO 9625410A1 (corresponding to Kokai Tokkyo Koho H9-183769).

According to the present invention, imidazolone and imidazolidinone derivatives having an excellent anti-fungal activity can be obtained in high purity and in good yield.

The following Reference Examples and Examples illustrate the present invention in detail, but are not to be construed to limit the scope thereof.

¹H-NMR spectrum is measured by a Varian Jemini 200 (200 MHz) type spectrometer using tetramethylsilane as an internal standard, and all δ values were represented by ppm. The numerical value described in ( ) for the mixed solvent is a volume ratio of each solvent. 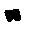 %*"* is by weight unless otherwise stated. A ratio of the solvent in silica gel chromatography is a volume ratio of solvents to be mixed.

The symbols in the Examples have the following meanings. s: singlet, d: doublet, t: triplet, q: quartet, dd: double doublet, tt: triple triplet, m: multiplet, br: broad, J: coupling constant.

### [Examples]

### Reference Example 1

A mixture of 4-fluoronitrobenzene (50.4 g),1H-tetrazole (25 g), potassium carbonate (50 g) and N,N-dimethylformamide (350ml) was stirred at 70 to 75°C for 10 hours. The reaction mixture was cooled, and water (2500ml) was added. The precipitated crystals were collected by filtration and washed with water (500ml).
Yellow crystals of a mixture of 1-(4-nitrophenyl)-1H-tetrazole and 2-(4-nitrophenyl)-2H-tetrazole obtained above was dissolved in a mixture of methanol (280ml)and tetrahydrofuran (420ml), and ferric chloride (0.33 g) and activated carbon (3.3 g) were added to the solution. To the mixture was added dropwise hydrazine monohydrate (27 g) under reflux over a period of 10 minutes. After the addition was complete, the mixture was stirred for 10 hours under reflux. The reaction mixture was cooled, and the activated carbon was filtered off. The activated carbon was washed with methanol (200ml). The filtrate and the washing were combined, and the solvent was distilled off under reduced pressure. The residue was subjected to silica gel chromatography (eluent : tetrahydrofuran*/*hexane= 2*/*3→ tetrahydrofuran*/*hexane*/* ethyl acetate =1*/*1*/*1). The first fraction was concentrated in vacuo, and the residue was crystallized from hexane to give 2-(4-aminophenyl)-2H-tetrazole (14.48 g) as pale yellow powdery crystals. The crystals were recrystallized from a mixture of ethyl acetate and diisopropyl ether.
Melting Point : 124-125°C
1H-NMR(d₆-DMSO)δ : 5.76(2H,s), 6.76(2H,d,J=8.8Hz), 7.74(2H,d,J=8.8Hz), 9.08(1H,s)

| Elemental Analysis: C₇H₇N₅ | | | |
|---|---|---|---|
| Calcd.(%) : | C, 52.17; | H, 4.38; | N, 43.45 |
| Found(%) : | C, 52.01; | H, 4.44; | N, 43.41 |

The second fraction was concentrated in vacuo, and the residue was crystallized from diisopropyl ether to give 1-(4-aminophenyl)-1H-tetrazole (25.29 g) as pale yellow powdery crystals. The crystals were recrystallized from a mixture of ethyl acetate and diisopropyl ether.
Melting Point 142-143°C
¹H-NMR(d₆-DMSO)δ : 5.65(2H,s), 6.73(2H,d,J=8.8Hz), 7.48(2H,d,J=8.8Hz), 9.83(1H,s)

| Elemental Analysis: C₇H₇N₅ | | | |
|---|---|---|---|
| Calcd.(%) : | C, 52.17; | H, 4.38; | N, 43.45 |
| Found(%) : | C, 51.88; | H, 4.38; | N, 43.62 |

### Reference Example 2

1-(4-aminophenyl)-1H-tetrazole (15.29 g) and pyridine (9 g) was dissolved in a mixture of tetrahydrofuran (200ml) and ethyl acetate (100ml). To the solution was added dropwise phenyl chlorocarbonate (17.6 g) with stirring under ice cooling. After the addition was complete, the reaction mixture was stirred at room temperature for 1 hour. To the reaction mixture were added water (100ml) and a saturated aqueous solution of sodium chloride (100ml). The organic layer was washed with water (100ml×2) and dried over anhydrous magnesium sulfate. The solvent was distilled off under reduced pressure. To the residue was added diisopropyl ether (500ml), and the resulting crystals were collected by filtration. The crystals were washed with diisopropyl ether (150ml) and dried to give phenyl 4-(1H-tetrazol-1-yl)phenylcarbamate (24.15 g) as colorless scaly crystals. The crystals were recrystallized from ethyl acetate.
Melting Point 186-189°C
¹H-NMR(d₆-DMSO)δ : 7.25-7.34(3H,m), 7.42-7.50(2H,m), 7.77(2H,d,J=9Hz), 7.89(2H,d,J=9Hz), 10.0(1H,s), 10.6(1H,s)

| Elemental Analysis: C₁₄H₁₁N₅O₂ | | | |
|---|---|---|---|
| Calcd.(%) : | C, 59.78; | H, 3.94; | N, 24.90 |
| Found(%) : | C, 59.56; | H, 3.99; | N, 24.89 |

### Reference Example 3

p-Nitroaniline (14.75 g) was dissolved in methanol (400ml). To the solution was added 2,2-dichloroacetoaldehyde p-toluenesulfonyl hydrazone (10.00 g) at -3--4°C, and the mixture was stirred for 1 hour. The mixture was further stirred at room temperature over night. The reaction mixture was concentrated under reduced pressure to make the volume of the mixture to about 170ml and allowed to stand under ice cooling for 30 minutes. The resulting crystals were collected by filtration, washed with diisopropyl ether and dried under reduced pressure to give 1-(4-nitrophenyl)-1H-1,2,3-triazole (4.04 g) as pale yellow crystals.
The mother liquor was concentrated and the residue was dissolved in a mixture of ethyl acetate (200ml) and tetrahydrofuran (200ml). The mixture was washed with 1N-hydrochloric acid (150ml), 1N-sodium hydroxide aqueous solution (150ml), water (150ml) and a saturated aqueous solution of sodium chloride (150ml), successively. The organic layer was dried over anhydrous magnesium sulfate, and the solvent was distilled off under reduced pressure. The residue was crystallized from diethyl ether to give the above compound (0.47 g).
Yield: 4.51 g, 67%
The product was recrystallized from a mixture of dichloromethane and diisopropyl ether.
Melting Point 205-206°C

### Reference Example 4

To an ice-cooled mixture of 40% aqueous solution of glyoxal (23.4ml), acetic acid (1ml) and methanol (300ml) was added p-nitroaniline (22.25 g), and the mixture was stirred for 1 hour. To the mixture was added p-toluenesulfonyl hydrazine (10.00 g), and the mixture was stirred at 0°C for 2 hours and at room temperature overnight. The solvent was concentrated under reduced pressure. To the residue was added ethyl acetate (500ml), and the mixture was extracted under reflux. The extract was washed with 0.5N sodium hydroxide aqueous solution (250ml), water (200ml) and a saturated aqueous solution of sodium chloride (200ml), successively, and dried over anhydrous magnesium sulfate. The solvent was distilled off under reduced pressure to make the volume of solution to about 100ml, and the concentrate was kept at room temperature overnight. The resulting crystals were collected by filtration to give 1-(4-nitrophenyl)-1H-1,2,3-triazole (5.31 g) as pale yellow crystals.
The mother liquor was concentrated, and the residue was purified by silicagel column chromatography (eluent : ethyl acetate*/*hexane =1*/*2→1*/*1). Crystallization from ethyl acetate gave the above compound (2.17 g).
Yield: 7.48 g, 73%

### Reference Example 5

A mixture of 1-(4-nitrophenyl)-1H-1,2,3-triazole (33.33 g), 10% palladium-carbon (50% wet, 4.00 g), ethanol (200ml) and tetrahydrofuran (200ml) was stirred at 50°C for 6 hours under a hydrogen atmosphere. After the reaction mixture was cooled, the catalyst was filtered off, and the solvent was distilled off under reduced pressure. The residue was crystallized from diisopropyl ether to give 1-(4-aminophenyl)-1H-1,2,3-triazole (26.45 g, 94%) as pale yellow crystals. The product was recrystallized from ethyl acetate.
Melting Point 121-122°C
¹H-NMR(CDCl₃)δ : 3.93(2H,bs), 6.77(2H,dt,J=9Hz,2.2Hz), 7.48(2H,dt,J=9Hz,2Hz), 7.81(1H,s), 7.87(1H,s)

| Elemental Analysis: C₈H₈N₄ | | | |
|---|---|---|---|
| Calcd.(%) : | C, 59.99; | H, 5.03; | N, 34.98 |
| Found(%) : | C, 60.02; | H, 5.08; | N, 34.66 |

### Reference Example 6

To a mixture of 1-(4-nitrophenyl)-1H-1,2,3-triazole (26.45 g), pyridine (14.40 g) and acetone (550ml) was added dropwise phenyl chlorocarbonate (28.50 g) under ice cooling over a period of 15 minutes. After the addition was complete, the reaction mixture was stirred at room temperature for 1 hour. The solvent was distilled off under reduced pressure. To the residue were added ethyl acetate (400ml), tetrahydrofuran (200ml) and water (300ml). The organic layer was separated, washed with water (200ml) and a saturated aqueous solution of sodium chloride (200ml), successively and dried over anhydrous magnesium sulfate. The solvent was distilled off under reduced pressure. The residue was crystallized from diisopropyl ether to give phenyl 4-(1H-1,2,3-triazole-1-yl)phenylcarbamate (39.68 g) as colorless powdery crystals.
Yield 85%
The product was recrystallized from ethyl acetate.
¹H-NMR(d₆-DMSO)δ : 7.24-7.49(5H,m), 7.72(2H,d,J=9Hz), 7.88(2H,d,J=9Hz), 7.96(1H,s), 8.76(1H,s), 10.53(1H,br)

| Elemental Analysis: C₁₅H₁₂N₄O₂ | | | |
|---|---|---|---|
| Calcd.(%) : | C, 64.28; | H, 4.31; | N, 19.98 |
| Found(%) : | C, 64.38; | H, 4.32; | N, 20.02 |

### Reference Example 7

4-Fluoronitrobenzene (70.6 g) was dissolved in N,N-dimethylformamide (490ml). To the solution were added 1H-tetrazole (35 g) and potassium carbonate (70 g), and the mixture was stirred at 75°C for 9.5 hours. After having been cooled, the reaction mixture was poured into ice water (3000ml). The resulting solid was collected by filtration and washed with water (500ml×3) to give a mixture of 1-(4-nitrophenyl)-1H-tetrazole and 2-(4-nitrophenyl)-2H-tetrazole as a white powder. The product was added to a mixture of tetrahydrofuran (500ml) and methanol (100ml). To the mixture were added activated carbon (powder, 12 g) and anhydrous ferric chloride (FeCl₃, 1.2 g), and the mixture was heated at 75°C. To the mixture was added dropwise hydrazine monohydrate (70ml) over a period of 2 hours. After the addition was complete, the mixture was stirred at 75°C for 6 hours. The reaction mixture was cooled and filtered. The activated carbon was washed with ethyl acetate (100ml×2). The filtrate and washings were combined and washed with a saturated aqueous solution of sodium chloride (100ml, 50ml×2) to give a solution containing 1-(4-aminophenyl)-1H-tetrazole and 2-(4-aminophenyl)-2H-tetrazole.
To the solution were added water (200ml) and sodium hydrogencarbonate (42 g). To the stirred mixture was added dropwise phenyl chlorocarbonate (50ml) over a period of 30 minutes at room temperature. After the addition was complete, the reaction mixture was stirred for 15 minutes, and the organic layer was separated. The organic layer was washed with a saturated aqueous solution of sodium chloride (100ml×2), dried over anhydrous magnesium sulfate and concentrated under reduced pressure to make the quantity of the solution to about 200 g. To the concentrates were added ethyl acetate (100ml), and diisopropyl ether (100ml) and the mixture was allowed to stand. The precipitated crystals were collected by filtration and washed with a mixture of ethyl acetate and diisopropyl ether (1 : 1, 100ml, 50ml). To the crystals was added tetrahydrofuran (400ml), and the mixture was heated to dissolve the crystals. The solution was concentrated under reduced pressure to make the quantity to 200 g. To the concentrate was added a mixture of ethyl acetate and diisopropyl ether (1 : 1 : 200ml), and the mixture was allowed to stand. The resulting crystals were collected by filtration and washed with ethyl acetatediisopropyl ether (1 : 1 : 100ml, 50ml). The recrystallization was repeated twice in the same manner as described above to give phenyl 4-(1H-tetrazol-1-yl)phenylcarbamate (39 g) as colorless crystals. This product was identical with the compound obtained in Reference Example 2 in physicochemical properties.

### Reference Example 8

By a manner similar to that shown in Chemistry of Heterocyclic Compounds, Vol. 21, p 1257 (1985), p-nitroaniline (138 g) was converted to 1-(4-nitrophenyl)-1H-tetrazole. The product was dissolved in tetrahydrofuran (2800ml) under heating at 60°C. To the solution were added anhydrous ferric chloride (1.8 g) and activated carbon (powder, 19 g). The mixture was refluxed gently, and hydrazine · monohydrate (180 g) was added dropwise over a period of 30 minutes with stirring. After the addition was complete, the mixture was stirred for 2.5 hours under reflux. After the mixture had been cooled, the insoluble substance was filtered off. The insoluble substance was washed with ethyl acetate (1400ml). The filtrate and the washing were combined and washed with a saturated aqueous solution of sodium chloride (1500ml×2) to give a solution containing 1-(4-aminophenyl)-1H-tetrazole. To the solution was added pyridine (95 g). To the mixture was added dropwise phenyl chlorocarbonate (188 g) over a period of 15 minutes with stirring under ice cooling. After the addition was complete, the mixture was stirred at room temperature for 1 hour, and a saturated aqueous solution of sodium chloride (1500ml) was added. The organic layer was separated and washed with water (1500ml×2). The solvent was distilled off under reduced pressure. To the residue was added diisopropyl ether (2000ml) and the mixture was stirred. The resulting crystals were collected by filtration to give phenyl 4-(1H-tetrazol-1-yl)phenylcarbamate (230.9 g). This product was identical with the compound obtained in Reference Example 2 in physicochemical properties.

### Reference Example 9

To a solution of p-nitroaniline (10g) in N,N-dimethylformamide (50 ml) were added benzyl bromide (49.5 g) and potassium carbonate (22 g), and the mixture was stirred at 160°C for 32 hours. After having been cooled to room temperature, the reaction mixture was poured into ice-water (200 ml) and extracted with ethyl acetate (300 ml). The organic layer was washed with water (300 ml x 3) and a saturated aqueous solution of sodium chloride (300 ml) and dried over anhydrous sodium sulfate. The solvent was distilled off under reduced pressure. To the residue were added hexane (100 ml) and diisopropyl ether (100 ml). The resulting crystals were collected by filtration. The crystals were washed with diisopropyl ether (50 ml) and dried under reduced pressure to give N,N-dibenzyl-p-nitroaniline (19 g) as pale yellow crystals.
¹H-NMR(CDCl₃)δ : 4.76 (4H, s), 6.70 (2H, d, J=9.2Hz), 7.81-7.41 (10H, m), 8.07 (2H, d, J=9.2Hz).

### Reference Example 10

To a solution of N,N-dibenzyl-p-nitroaniline (18 g) in methanol (120 ml) were added activated carbon (2.2 g) and ferric chloride (0.55 g), and the mixture was stirred for 30 minutes under reflux. To the reaction mixture was added dropwise hydrazine monohydrate (27.4 ml) under reflux over a period of 1 hour, and the mixture was refluxed for 24 hours. The reaction mixture was cooled to room temperature, and activated carbon (2.2 g) and ferric chloride (0.55 g) were added. The mixture was refluxed for 10 minutes, and hydrazine monohydrate (27.4 ml) was added dropwise under reflux over a period of 1 hour. The mixture was refluxed further for 24 hours. After the mixture had been cooled, activated carbon was filtered off, and the solvent was distilled off under reduced pressure. The residue was dissolved in ethyl acetate (130 ml) and washed with water (100 ml x 3) and a saturated aqueous solution of sodium chloride (100 ml), successively. The ethyl acetate layer was dried over anhydrous sodium sulfate and concentrated under reduced pressure. To the residue was added a mixture of hexane and diisopropyl ether (3 : 1, 24 ml) to deposit crystals. The resulting crystals were collected by filtration and dried under reduced pressure to give N,N-dibenzyl-p-phenylenediamine (15g) as purple crystals.
¹H-NMR(CDCl₃)δ : 3.29 (2H, brs), 4.51 (4H, s), 6.57 (2H, d, J=8.4Hz), 6.64 (2H, d, J=8.4Hz), 7.18-7.34 (10H, m).

### Reference Example 11

To a mixture of N,N-dibenzyl-p-phenylenediamine (15g), ethyl orthoformate (25.5 ml) and sodium aside (4 g) was added dropwise acetic acid (40 ml) at room temperature over a period of 10 minutes. The mixture was stirred at room temperature under a nitrogen atmosphere for 5 minutes and for a further 4 hours at 90°C. After the reaction mixture was cooled to room temperature, water (50 ml) and 6N sodium hydroxide aqueous solution (18 ml) were added. To the mixture was added a solution of sodium nitrite (2 g) in water (8.4 ml). The mixture was stirred under ice cooling for 10 minutes. The resulting powder was collected by filtration, washed with water (120 ml) and diisopropyl ether (30 ml) and dried under reduced pressure. The resulting brown powder was recrystallized from a mixture of ethanol (350 ml), methanol (10 ml) and THF (1 ml) to give 1-[4-(N,N-dibenzylamino)phenyl]-1H-tetrazole (12.4 g) as brown scaly crystals.
¹H-NMR(CDCl₃)δ : 4.74 (4H, s), 6.82 (2H, d, J=9.2Hz), 7.23-7.43 (12H, m), 8.80(1H, s).

### Reference Example 12

To a solution of 1-[4-(N,N-dibenzylamino)phenyl]-1H-tetrazole (30 mg) in methanol (1 ml) were added 10% palladium-carbon (50 mg) and 6N-sodium hydroxide aqueous solution (0.015 ml), and the mixture was stirred at room temperature for 5 hours under a hydrogen atmosphere. The reaction mixture was filtered, and to the filtrate was added water (1 ml). To the mixture was added dropwise 1N sodium hydroxide aqueous solution (0.11 ml) under ice cooling. The mixture was extracted with ethyl acetate (10 ml), and the organic layer was washed with a saturated aqueous solution of sodium chloride (20 ml x 3) and dried over anhydrous sodium sulfate. The solvent was distilled off under reduced pressure to give 1-(4-aminophenyl)-1H-tetrazole (11 mg) as a brown powder. This product was identical with 1-(4-aminophenyl)-1H-tetrazole obtained in Reference Example 1 in physicochemical properties.

### Reference Example 13

To a solution of 1-[4-(N,N-dibenzylamino)phenyl]-1H-tetrazole (2 g) in methanol-THF(2 : 1, 30 ml) were added 10% palladium-carbon (0.5 g) and 6N sodium hydroxide aqueous solution (1 ml), and the mixture was stirred at room temperature for 25 minutes under a hydrogen atmosphere. The reaction mixture was filtered, and the filtrate was concentrated under reduced pressure. To the residue were added a mixture of ethyl acetate and THF(2 : 1, 45 ml) and water (5 ml). To the mixture was added dropwise a solution of sodium hydrogencarbonate (1.3 g) in water (20 ml) under ice cooling over a period of 5 minutes. To the mixture was added dropwise phenyl chlorocarbonate (0.89 ml) at room temperature over a period of 5 minutes. The mixture was stirred at room temperature for 20 minutes. To the mixture was added water (10 ml). The mixture was stirred further for 30 minutes. The organic layer was washed with a saturated aqueous solution of sodium chloride (10 ml x 3), dried over anhydrous sodium sulfate, and the solvent was distilled off under reduced pressure to make the quantity to 6.6 g. To the residue was added diisopropyl ether (15 ml). The resulting crystals were collected by filtration and dried under reduced pressure to give phenyl 4-(1H-tetrazol-1-yl)phenylcarbamate (1.5 g) as pale brown crystals. This product was identical with the compound obtained in Reference Example 2 in physicochemical properties.

### Working Example 1

(2R,3S)-2-(2,4-Difluorophenyl)-3-methyl-2-(1H-1,2,4-triazol-1-yl)methyloxilane (40g) was dissolved in 1-propanol (400ml), and to the solution was added titanium tetraisopropoxide (68g). To the mixture was added 2,2-diethoxyethylamine (464ml), and the mixture was refluxed with heating under a nitrogen atmosphere for 24 hour. The reaction mixture was cooled, and the solvent and 2,2-diethoxyethylamine were distilled off under reduced pressure. The residue was dissolved in ethyl acetate (800ml), and to the solution was added a mixture of 1N-aqueous solution of sodium hydroxide (260ml) and saturated aqueous solution of sodium chloride (260ml) with stirring. The mixture was stirred for 1 hour at room temperature.
The resulting insoluble substance was filtered off, and the substance was washed with ethyl acetate (250ml). The filtrate and the washing were combined, and the ethyl acetate layer was separated. The ethyl acetate layer was extracted with 1N-hydrochloric acid (150ml×4). The hydrochloric acid layers were combined and neutralized with 2N-sodium hydroxide solution (300ml) under ice cooling. The resulting mixture was extracted with ethyl acetate (400ml×2). The ethyl acetate layers were combined, washed with 1N-sodium hydroxide solution (200ml) and saturated aqueous solution of sodium chloride (200ml) successively, and dried over anhydrous magnesium sulfate. The solvent was distilled off under reduced pressure to give (2R,3R)-3-(2,2-diethoxyethyl)amino-2-(2,4-difluorophenyl)-1-(1H-1,2,4-triazol-1-yl)-2-butanol (49.4g) as a pale yellow oily substance.
Yield : 73%
¹H-NMR(CDCl₃)δ : 0.91(3H,d,J=6.6Hz), 1.0-1.2(1H,br), 1.23(6H,t,J=7Hz), 2.66(1H,dd,J=12Hz,4.6Hz), 2.95(1H,dd,J=12Hz,6.4Hz), 3.14(1H,q,J=6.6Hz), 3.48-3.81(4H,m), 4.56(1H,dd,J=6.4Hz,4.6Hz), 4.75(1H,d,J=14Hz), 4.88(1H,s), 4.89(1H,d,J=14Hz), 6.69-6.80(2H,m), 7.33-7.45(1H,m), 7.76(1H,s), 7.93(1H,s)

### Working Example 2

(2R,3S)-2-(2,4-Difluorophenyl)-3-methyl-2-(1H-1,2,4-triazol-1-yl)methyloxilane (10.0g) was dissolved in ethanol (100ml), and to the solution were added titanium tetraisopropoxide (17.0g) and 2,2-diethoxyethylamine (106g) successively. The mixture was heated for 40 hours under an argon atmosphere under reflux. The reaction mixture was cooled, and the solvent and 2,2-diethoxyethylamine were distilled off under reduced pressure. The residue was dissolved in ethyl acetate (200ml), and to the solution was added a mixture of 1N-sodium hydroxide solution (65ml) and saturated aqueous solution of sodium chloride (65ml) with stirring. The mixture was stirred for 1 hour at room temperature. The resulting insoluble substance was filtered off, and the substance was washed with ethyl acetate (50ml). The filtrate and the washing were combined, and the ethyl acetate layer was separated. The ethyl acetate layer was extracted with 1N-hydrochloric acid (150ml). The hydrochloric acid layer was neutralized with 1N-sodium hydroxide solution (150ml) under ice cooling, and the mixture was extracted with ethyl acetate (300ml). The ethyl acetate layer was washed with saturated aqueous solution of sodium chloride (100ml) and dried over anhydrous magnesium sulfate. The solvent was distilled off under reduced pressure. The residue was subjected to silica gel column chromatography [silica gel 500g, eluent (acetone : hexane=1 : 2)] to give (2R,3R)-3-(2,2-diethoxyethyl)amino-2-(2,4-difluorophenyl)-1-(1H-1,2,4-triazol-1-yl)-2-butanol (12.4g) as a pale yellow oily substance.
Yield : 81%

### Working Example 3

(2R,3S)-2-(2-Fluorophenyl)-3-methyl-2-(1H-1,2,4-triazol-1-yl)methyloxilane (10.0g) was dissolved in 1-propanol (100ml). To the solution were added titanium tetraisopropoxide (183g) and 2,2-diethoxyethylamine (114g) successively, and the mixture was heated for 24 hours under a nitrogen atmosphere under reflux. The reaction mixture was cooled, and the solvent and 2,2-diethoxyethylamine were distilled off under reduced pressure. The residue was dissolved in ethyl acetate (200ml) and to the solution was added a mixture of 1N-sodium hydroxide solution (65ml) and saturated aqueous solution of sodium chloride (65ml) with stirring. The mixture was stirred for 1 hour at room temperature. The resulting insoluble substance was filtered off, and substance was washed with ethyl acetate (100ml). The filtrate and washing were combined, and the ethyl acetate layer was separated. The ethyl acetate layer was extracted with 1N-hydrochloric acid (200ml,100ml). The hydrochloric layers are combined and neutralized with 2N-sodium hydroxide solution (150ml) under ice cooling. The mixture was extracted with ethyl acetate (200ml,100ml). The ethyl acetate layers were combined, washed with 1N-sodium hydroxide solution (100ml) and saturated aqueous solution of sodium chloride (100ml) successively and dried over anhydrous magnesium sulfate.
The solvent was distilled off under reduced pressure to give (2R,3R)-3-(2,2-diethoxyethyl)amino-2-(2-fluorophenyl)-1-(1H-1,2,4-triazol-1-yl)-2-butanol (13.6g) as a pale yellow oily substance.
Yield : 87%
¹H-NMR(CDCl₃)δ : 0.92(3H,d,J=6.6Hz), 1.0-1,2(1H,br), 1.23(6H,t,J=7Hz), 2.67(1H,dd,J=12Hz,4.6Hz), 2.95(1H,dd,J=12Hz,6.4Hz), 3.14(1H,q,J=6.6Hz), 3.49-3.80(4H,m), 4.56(1H,dd,J=6.4Hz,4.6Hz), 4.79(1H,d,J=14Hz), 4.84(1H,s), 4.94(1H,d,J=14Hz), 6.93-7.45(4H,m), 7.76(1H,s), 7.89(1H,s)

### Working Example 4

(2R,3R)-3-(2,2-Diethoxyethyl)amino-2-(2,4-difluorophenyl)-1-(1H-1,2,4-triazol-1-yl)-2-butanol (0.64g) was dissolved in ethanol (6.4ml), and to the solution were added 4-(2,2,3,3-tetrafluoropropoxy)phenylcarbamic acid phenyl ester (1.13g) and pyridine (0.14ml). The mixture was heated for 18 hours under reflux. The reaction mixture was cooled and concentrated under reduced pressure. To the residue were added ethyl acetate (30ml) and water (30ml). The ethyl acetate layer was separated, washed with 10% aqueous solution of phosphoric acid (30ml) and saturated aqueous solution of sodium chloride (30ml) successively and dried over anhydrous magnesium sulfate. The solvent was distilled off under reduced pressure. The residue was purified by silica gel column chromatography (eluent : ethyl acetate/hexane=2/1) and recrystallized from diisopropyl ether to give 1-(2,2-diethoxyethyl)-1-[(1R,2R)-2-(2,4-difluorophenyl)-2-hydroxy-1-methyl-3-(1H-1,2,4-triazol-1-yl)propyl]-3-[4-(2,2,3,3-tetrafluoropropoxy)phenyl]urea (0.72g) as colorless powdery crystals.
Yield : 69%
Melting point : 148-150°C

| Elemental analysis : C₂₈H₃₃F₆N₅O₅ | | | |
|---|---|---|---|
| Calcd. (%) : | C,53.08; | H,5.25; | N,11.05 |
| Found (%) : | C,53,14; | H,5.09; | N,11.14 |

¹H-NMR(CDCl₃)δ : 1.05(1.2H,d,J=7.2Hz), 1.21(1.8H,d,J=6.8Hz), 1.28-1.37(6H,m), 3.48-4.02(6.6H,m), 4.26-4.54(3H,m), 4.71(0.6H,m), 4.93-5.00(1H,m), 5.21-5.42(1.2H,m), 6.06(1H,tt,J=53Hz,5.0Hz), 6.69-6.93(4H,m), 7.25-7.42(2.6H,m), 7.49-7.79(2.4H,m), 8.24(0.6H,s), 8.43(0.6H,br), 8.69(0.4H,s)

### Working Example 5

1-(2,2-Diethoxyethyl)-1-[(1R,2R)-2-(2,4-difluorophenyl)-2-hydroxy-1-methyl-3-(1H-1,2,4-triazol-1-yl)propyl]-3-[4-(2,2,3,3-tetrafluoropropoxy)phenyl]urea (0.63g) was dissolved in a mixture of methanol (25ml) and water (5ml). To the solution was added 1N-hydrochloric acid (1ml), and the mixture was stirred for 24 hours at 50°C. The reaction mixture was cooled, and to the mixture was added 1N-sodium hydroxide solution (1ml). The mixture was concentrated under reduced pressure. To the residue were added ethyl acetate (40ml) and water (40ml). The ethyl acetate layer was separated, washed with water (40ml) and saturated aqueous solution of sodium chloride (40ml) successively , and dried over anhydrous magnesium sulfate.
The solvent was distilled off under reduced pressure. The residue was purified by silica gel column chromatography (eluent : ethyl acetate/hexane=2/1) and recrystallized from a mixture of ethyl acetate and diisopropyl ether to give 1-[(1R,2R)-2-(2,4-difluorophenyl)-2-hydroxy-1-methyl-3-(1H- 1,2,4-triazol-1-yl)propyl]-3-[4-(2,2,3,3-tetrafluoropropoxy)phenyl]-2(1H,3H)-imidazolone (0.40g) as colorless powdery crystals.
Yield : 74%
¹H-NMR(CDCl₃)δ : 1.20(3H,d,J=7Hz), 4.20(1H,d,J=14Hz), 4.38(2H,t,J=12Hz), 4.95(1H,q,J=7Hz), 5.10(1H,d,J=14Hz), 5.50-5.75(1H,br), 6.07(1H,tt, J=53Hz,5Hz), 6.60(1H,d,J=3Hz), 6.73(1H,d,J=3Hz), 6.74-6.84(2H,m), 7.02 (2H,d,J=9Hz), 7.43-7.56(1H,m), 7.59(2H,d,J=9Hz), 7.73(1H,s), 7.86(1H,s)

### Working Example 6

(2R,3R)-3-(2,2-Diethoxyethyl)amino-2-(2-fluorophenyl)-1-(1H-1,2,4-triazol-1-yl)-2-butanol (13.0g) was dissolved in dimethylsulfoxide (40ml). To the solution was added 4-(2,2,3,3-tetrafluoropropoxy)phenylcarbamic acid phenyl ester (12.2g). The mixture was stirred for 3 hours at 110°C. The reaction mixture was cooled, diluted with ethyl acetate (200ml) and washed with a diluted aqueous solution of sodium chloride (220ml×2). The ethyl acetate layer was washed with 0.5N-hydrochloric acid (150ml), a diluted aqueous solution of sodium chloride (150ml), 0.5N-aqueous solution of sodium hydroxide (150×2ml), a diluted aqueous solution of sodium chloride (150ml) and saturated aqueous solution of sodium chloride (150ml) successively , and dried over anhydrous magnesium sulfate. The solvent was distilled off under reduced pressure. The residue was recrystallized from a mixture of ethyl acetate and diisopropyl ether to give 1-(2,2-diethoxyethyl)-1-[(1R,2R)-2-(2-fluorophenyl)-2-hydroxy-1-methyl-3-(1H-1,2,4-triazol-1-yl)propyl]-3-[4-(2,2,3,3-tetrafluoropropoxy)phenyl]urea (13.0g) as colorless powdery crystals. The mother liquor was concentrated, and the residue was purified by silica gel column chromatography (eluent : ethyl acetate/hexane=1/1) and recrystallized from diisopropyl ether to give the above compound (1.8g) as colorless powdery crystals.
Total yield : 68%
Melting point 133-134°C

| Elemental analysis : C₂₈H₃₄F₅N₅O₅ | | | |
|---|---|---|---|
| Calcd. (%) : | C,54.63; | H,5.57; | N,11.38 |
| Found (%) : | C,54,53; | H,5.49; | N,11.41 |

¹H-NMR (CDCl₃)δ : 1.05(1.2H,d,J=7.4Hz), 1.21(1.8H,d,J=6.6Hz), 1.28-1.37(6H,m), 3.50-4.05(6.6H,m), 4.32(2H,t,J=12Hz), 4.37(0.4H,d,J=14Hz), 4.55(0.6H,d,J=14Hz), 4.71(0.6H,m), 4.96-5.10(1.4H,m), 5.31-5.50(0.8H,m), 6.07(1H,tt,J=53Hz,5.0Hz), 6.86-7.06(4H,m), 7.15-7.42(3.4H,m), 7.54-7.66(1.8H,m), 7.76(0.8H,s), 8.23(0.6H,s), 8.43(0.6H,br), 8.70(0.4H,s)

### Working Example 7

1-(2,2-Diethoxyethyl)-1-[(1R,2R)-2-(2-fluorophenyl)-2-hydroxy-1-methyl-3-(1H-1,2,4-triazol-1-yl)propyl]-3-[4- (2,2,3,3-tetrafluoropropoxy)phenyl]urea (12.8g) was dissolved in methanol (130ml), and to the solution was added 2N-hydrochloric acid (104ml). The mixture was heated for 2 hours under reflux. The reaction mixture was cooled, and to the reaction mixture was added sodium hydrogencarbonate (17.5g). The mixture was concentrated under reduced pressure. To the residue were added ethyl acetate (200ml) and water (200ml). The ethyl acetate layer was separated, washed with water (100ml), saturated aqueous solution of sodium chloride (100ml) successively, and dried over anhydrous magnesium sulfate. The solvent was distilled off under reduced pressure. The residue was recrystallized from a mixture of ethyl acetate and diisopropyl ether to give 1-[(1R,2R)-2-(2-fluorophenyl)-2-hydroxy-1-methyl-3-(1H-1,2,4-triazol-1-yl)propyl]-3-[4-(2,2,3,3-tetrafluoropropoxy)phenyl]-2(1H,3H)-imidazolone (9.85g) as colorless powdery crystals.
Yield : 91%

| Elemental analysis : C₂₄H₂₂F₅N₅O₃ | | | |
|---|---|---|---|
| Calcd. : | C,55.07; | H,4.24; | N,13.38 |
| Found (%) : | C,54,98; | H,4.03; | N,13.55 |

¹H-NMR(CDCl₃)δ : 1.20(3H,d,J=7Hz), 4.21(1H,d,J=14Hz), 4.37(2H,t,J=12Hz), 5.03(1H,q,J=7Hz), 5.15(1H,d,J=14Hz), 5.42(1H,br), 6.07(1H,tt,J=53Hz,4.8Hz), 6.59(1H,d,J=3.2Hz), 6.76(1H,d,J=3.2Hz), 6.97-7.07(2H,m), 7.01(2H,d,J=9Hz), 7.17-7.29(1H,m), 7.43-7.51(1H,m), 7.59(2H,d,J=9Hz), 7.72(1H,s), 7.81(1H,s)

### Working Example 8

(2R,3R)-3-(2,2-Diethoxyethyl)amino-2-(2,4-difluorophenyl)-1-(1H-1,2,4-triazol-1-yl)-2-butanol (188mg) and 4-(1H-1,2,3-triazoll-yl)phenylcarbamic acid phenyl ester (137mg) were added to dimethylformamide (2ml), and the mixture was heated with stirring for 2 hours at 110 °C under an argon atmosphere.
The reaction mixture was cooled and poured into water (20ml). The mixture was extracted with ethyl acetate (20ml,15ml×2). The extracts were combined, washed with water (30ml) and saturated aqueous solution of sodium chloride (30ml) successively, and dried over anhydrous magnesium sulfate. The solvent was distilled off under reduced pressure. The residue was purified by silica gel column chromatography (eluent : ethyl acetate/hexane=3/1→ethyl acetate) to give 1-(2,2-diethoxyethyl)-1-[(1R,2R)-2-(2,4-difluorophenyl)-2-hydroxy-1-methyl-3-(1H-1,2,4-triazol-1-yl)propyl]-3-[4-(1H-1,2,3-triazol-1-yl)phenyl]urea (262mg) as white powdery crystals.
Yield : 94%

| Elemental analysis : C₂₇H₃₂F₂N₈O₄ | | | |
|---|---|---|---|
| Calcd. (%) : | C,56.83; | H,5.65; | N,19.64 |
| Found (%) : | C,56,71; | H,5.76; | N,19.44 |

¹H-NMR (CDCl₃)δ : 1.07(1.2H,d,J=7.0Hz), 1.23(1.8H,d,J=7.0Hz), 1.34(6H,t,J=7.0Hz),3.51-4.07(7H,m),4.39(0.4H,d,J=14Hz), 4.54(0.6H,d,J=14Hz), 4.74(0.6H,m), 4.94-5.01(1H,m), 5.24-5.41(1.4H,m), 6.72-6.82(2H,m), 7.37-7.84(8.2H,m), 7.95(0.6H,s), 8.24(0.4H,s), 8.75(0.4H,br), 9.05(0.4H,s)

### Working Example 9

1-(2,2-Diethoxyethyl)-1-[(1R,2R)-2-(2,4-difluorophenyl)-2-hydroxy-1-methyl-3-(1H-1,2,4-triazol-1-yl)propyl]-3-[4-(1H-1,2,3-triazol-1-yl)phenyl]urea (228mg) was dissolved in methanol (2.4ml). To the solution was added 2N-hydrochloric acid (2.4ml), and the mixture was heated at 60°C for 4 hours. The reaction mixture was cooled and neutralized with 1N-aqueous solution of sodium hydroxide (4.8ml). The methanol was distilled off under reduced pressure, and the residue was extracted with ethyl acetate (20ml,10ml×2). The extracts were combined, washed with water (20ml) and saturated aqueous solution of sodium chloride (20ml) successively, and dried over anhydrous magnesium sulfate. The solvent was distilled off under reduced pressure. The residue was crystallized from a mixture of ethyl acetate and diisopropyl ether to give 1-[(1R,2R)-2-(2,4-difluorophenyl)-2-hydroxy-1-methyl-3-(1H-1,2,4-triazol-1-yl)propyl]-3-[4-(1H-1,2,3-triazol-1-yl)phenyl]-2(1H,3H)-imidazolone (161mg) as colorless powdery crystals.
Yield : 84%

| Elemental analysis : C₂₃H₂₀F₂N₈O₂ | | | |
|---|---|---|---|
| Calcd. (%) : | C,57.74; | H,4.21; | N,23.42 |
| Found (%) : | C,57,47; | H,4.25; | N,23.48 |

¹H-NMR(CDCl₃)δ :1.22(3H,d,J=7Hz), 4.22(1H,d,J=14.4Hz), 5.01(1H,q,J=7Hz), 5.12(1H,d,J=14.4Hz), 5.49(1H,br), 6.72-6.86(4H,m), 7.42-7.50(1H,m), 7.75 (1H,s), 7.81-7.91(6H,m), 8.02(1H,s)

### Working Example 10

(2R,3R)-3-(2,2-Diethoxyethyl)amino-2-(2,4-difluorophenyl)-1-(1H-1,2,4-triazol-1-yl)-2-butanol (30g) was dissolved in N,N-dimethylformamide (300ml), and to the solution was added 4-(1H-tetrazol-1-yl)phenylcarbamic acid phenyl ester (21.93g). The mixture was stirred at 80°C for 5 hours. The reaction mixture was cooled, poured into water (300ml) and extracted with ethyl acetate (300ml×2). The ethyl acetate layer was washed with water (200ml×3) and dried over anhydrous magnesium sulfate. The solvent was distilled off under reduced pressure. The residue was recrystallized from a mixture of ethyl acetate and diisopropyl ether to give 1-(2,2-diethoxyethyl)-1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]urea (35.7g) as colorless powdery crystals. The mother liquor was concentrated and the residue was purified by silica gel column chromatography (eluent : ethyl acetate/hexane=3/1→ethyl acetate) and recrystallised from diisopropyl ether to give the above compound (4g) as colorless powdery crystals.
Total amount yielded 39.7g (Yield : 89%)
Melting point 180-182°C

| Elemental analysis : C₂₆H₃₁F₂N₉O₄ | | | |
|---|---|---|---|
| Calcd. (%) : | C,54.63; | H,5.47; | N,22.05 |
| Found (%) : | C,54,58; | H,5.37; | N,21.61 |

¹H-NMR(CDCl₃)δ : 1.08(1.5H,d,J=7Hz), 1.24(1.5H,d,J=7Hz), 1.31-1.38(6H,m), 3.50-4.06(7H,m), 4.39(0.5H,d,J=14Hz), 4.59(0.5H,d,J=14Hz), 4.76(0.5H,m), 4.93-5.08(1.5H,m), 5.25-5.48(1H,m), 6.73-6.83(2H,m), 7.31-7.69(5.5H,m), 7.79(0.5H,s), 7.82(0.5H,s), 8.24(0.5H,s), 8.85(0.5H,br), 8.94(0.5H,s), 8.96(0.5H,s), 9.16(0.5H,s)

### Working Example 11

1-(2,2-Diethoxyethyl)-1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]urea (39g) was dissolved in methanol (342ml), and to the solution was added 2N-hydrochloric acid (342ml). The mixture was stirred at 50 to 55°C for 17 hours. The reaction mixture was cooled, neutralized with 2N-NaOH (342ml) and then concentrated under reduced pressure. To the residue were added ethyl acetate (400ml) and water (400ml). The ethyl acetate layer was washed with water (200ml) and saturated aqueous solution of sodium chloride (200ml) successively , and dried over anhydrous magnesium sulfate.
The solvent was distilled off under reduced pressure. The residue was recrystallised from a mixture of ethyl acetate and diisopropyl ether to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-(1H,3H)-imidazolone (28.6g) as colorless powdery crystals.
Yield : 87%

| Elemental analysis : C₂₂H₁₉F₂N₉O₂ | | | |
|---|---|---|---|
| Calcd. (%) : | C,55.11; | H,3.99; | N,26.29 |
| Found (%) : | C,54,72; | H,4,04; | N,26.05 |

¹H-NMR(CDCl₃)δ : 1.22(3H,d,J=7Hz), 4.22(1H,d,J=14Hz), 5.03(1H,q,J=7Hz), 5.13(1H,d,J=14Hz), 5.45(1H,br), 6.74-6.88(4H,m), 7.42-7.54(1H,m), 7.76(1H,s), 7.82(2H,d,J=9Hz), 7.86(1H,s), 7.96(2H,d,J=9Hz), 9.06(1H,s)

### Working Example 12

A mixture of (2R,3S)-2-(2,4-difluorophenyl)-3-methyl-2-(1H-1,2,4-triazol-1-yl)methyloxilane (30g), 1-propanol (300ml), titanium tetraisopropoxide (51g) and 2-aminoethanol (109.4g) was stirred in a nitrogen stream for 7.5 hours under reflux. After the reaction mixture was cooled, ethyl acetate (500ml) was added. Then 2N-sodium hydroxide aqueous solution (98ml) saturated with sodium chloride was added to the mixture over a period of 3 minutes, and the mixture was stirred for 30 minutes. To the mixture was added saturated aqueous solution of sodium chloride (195ml), and the mixture was stirred for 10 minutes. The reaction mixture was allowed to stand, and the ethyl acetate layer was separated by decantation. To the aqueous layer was added ethyl acetate (400ml), and the mixture was stirred at room temperature for 3 minutes. The ethyl acetate layer was separated by decantation. The mixing with ethyl acetate followed by separation by decantation of the ethyl acetate layer was further repeated twice by using ethyl acetate (300ml each). The ethyl acetate layers were combined and washed with saturated aqueous solution of sodium chloride (250ml). The ethyl acetate layer was extracted with 1N-hydrochloric acid (225ml×2). The hydrochloric acid layers were combined, and to the acidic layer were added 2N-sodium hydroxide aqueous solution (230ml) saturated with sodium chloride to make the mixture alkaline. The mixture was extracted with ethyl acetate (300ml×2). The ethyl acetate layers were combined and washed with saturated aqueous solution of sodium chloride (200ml) and dried over anhydrous magnesium sulfate. The solvent was distilled off under reduced pressure. The residue was dried in vacuo to give (2R,3R)-2-(2,4-difluorophenyl)-3-(2-hydroxyethyl)amino-1-(1H-1,2,4-triazol-1-yl)-2-butanol (34.2g) as a pale yellow oily substance.
¹H-NMR(CDCl₃)δ : 0.92(3H,d,J=6.6Hz), 1.60-1.80(2H,br), 2.61-2.73(1H,m), 2.98-3.16(2H,m), 3.65-3.71(2H,m), 4.81(1H,d,J=14Hz), 4.96(1H,d,J=14Hz), 4.77-4.99(1H,br), 6.70-6.80(2H,m), 7.36-7.48(1H,m), 7.78(1H,s), 7.87 (1H,s)

### Working Example 13

To a solution of (2R,3R)-2-(2,4-difluorophenyl)-3-(2-hydroxyethyl)amino-1-(1H-1,2,4-triazol-1-yl)-2-butanol (34.2g) in ethyl acetate (600ml) were added 4-(1H-tetrazol-1-yl)phenylcarbamic acid phenyl ester (23.7g) and triethylamine (17.6ml), and the mixture was stirred for 90 minutes under reflux. After having been cooled, the mixture was stirred for 2 hours under ice cooling. The resulting crystals were collected by filtration, washed with ethyl acetate (50ml) and dried at 40°C under reduced pressure to give 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (37.9g) as colorless powdery crystals. The crystals included ethyl acetate.
¹H-NMR(d₆-DMSO)δ : 0.93,1.16(3H,d,J=7Hz); 3.47-4.09(4.5H,m); 4.56 (1H, d,J=14Hz); 4.91-5.03(1H,m); 5.30(0.5H,q,J=7Hz); 5.79, 6.12, 6.21 (2H,br); 6.87-6.96(1H,m); 7.13-7.45(2H,m); 7.59-7.88(5H,m); 8.21, 8.32 (1H,s); 9.41,9.49(1H,s); 10.02,10.05(1H,s)
SIMS (m/z) : 500(MH+)

### Working Example 14

(2R,3R)-2-(2,4-Difluorophenyl)-3-(2-hydroxyethyl)amino-1-(1H-1,2,4-triazol-1-yl)-2-butanol (6.6 g) was dissolved in N,N-dimethylformamide (132 ml), and to the solution was added 4-(1H-tetrazol-1-yl)phenylcarbamic acid phenyl ester (4.7g). The mixture was stirred at 50°C for 2.5 hours under nitrogen stream. The reaction mixture was cooled, poured into water (160 ml), and extracted twice with ethyl acetate (120 ml). The ethyl acetate layer was washed with water (160 ml×5), dried over anhydrous magnesium sulfate and concentrated under reduced pressure. The residue was dissolved in ethyl acetate and the solution was concentrated under reduced pressure. To the concentrate was added ethyl acetate (66 ml) to give 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (6.5 g) as white crystals. The product was identical with the compound obtained in Working Example 13 in physicochemical properties.

### Working Example 15

1-[(1R,2R)-2-(2,4-Difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (5.0 g) was dissolved in a mixture of N,N-dimethylacetamide (60 ml) and ethyl acetate (15 ml), and to the solution was added dropwise a solution of thionyl chloride (0.67 ml) in ethyl acetate (15 ml) over a period of 75 minutes under a nitrogen stream. The reaction mixture was stirred at 24 °C for 4 hours. Triethylamine (8.98 ml) was added dropwise to the mixture over a period of 10 minutes. The temperature of the reaction mixture was kept at 10 to 12°C, all through the addition of triethylamine. The reaction mixture was stirred for 1.5 hours at room temperature, poured into water (120 ml) and extracted with ethyl acetate (120 ml). The aqueous layer was separated and extracted with ethyl acetate (70 ml). The ethyl acetate layers were combined and washed with water (90 ml) three times, dried over anhydrous magnesium sulfate and evaporated under reduced pressure. The residue was dissolved in acetone (15 ml) and the solution was concentrated under reduced pressure. To the concentrate was added ethyl acetate (25 ml) to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-imidazolidinone (3.7 g) as white crystals.
¹H-NMR(CDCl₃)d : 1.08 (3H, d, J= 7 Hz), 3.74-4.11 (4H, m), 4.52 (1H, d, J= 14 Hz), 4.71 (1H, q, J= 7 Hz), 5.11 (1H, d, J= 14 Hz), 5.34 (1H, br), 6.73-6.84 (2H, m), 7.36-7.49 (1H, m), 7.69 (2H, d, J= 9 Hz), 7.77 (1H, s), 7.81 (2H, d, J= 9 Hz), 7.86 (1H, s), 8.97 (1H, s).

### Working Example 16

To a solution of 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (23.4 g) in N,N-dimethylacetamide-ethyl acetate (1 : 1, 240 ml) was added dropwise a solution of phosphorus oxychloride (6 ml) in ethyl acetate (70 ml) over a period of 2.5 hours at an internal temperature of 22 to 25°C, and then the mixture was stirred for 16 hours at room temperature. To the mixture was added dropwise triethylamine (60 ml) over a period of 2 hours at an internal temperature of -5 to -3°C, and then the mixture was stirred at room temperature for 3.5 hours. To the reaction mixture was added ice water (300 ml). The mixture was extracted with ethyl acetate (300 ml x 2). The extract was washed with water (300 ml), 5% aqueous solution of phosphoric acid (300 ml x 2), saturated aqueous solution of sodium hydrogencarbonate (300 ml) and saturated aqueous solution of sodium chloride (300 ml) successively. To the organic layer were added anhydrous magnesium sulfate and activated carbon (5 g), and the mixture was allowed to stand for 10 minutes, and filtered. The filtrate was concentrated and the residue was crystallized from ethyl acetate (50 ml). The crystals were collected by filtration and recrystallized from ethanol (600 ml). The crystals obtained were dried over phosphorus pentoxide for 1 hour at 40°C to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]- 2-imidazolidinone (11.1 g) as white crystals.
¹H-NMR(CDCl₃)δ : 1.08(3H,d,J=7Hz), 3.68-4.14(4H,m), 4.52(1H,d,J=14Hz), 4.71(1H,q,J=7Hz),5.12(1H,d,J=14Hz), 5.34(1H,br), 6.74-6.83(2H,m), 7.36-7.48(1H,m), 7.68(2H,d,J=9Hz), 7.77(1H,s), 7.81(2H,d,J=9Hz), 7.86 (1H,s), 8.96(1H,s).

| Elemental analysis : C₂₂H₂₁F₂N₉O₂ | | | |
|---|---|---|---|
| Calcd. (%) : | C,54.88; | H,4.40; | N,26.18. |
| Found (%) : | C,54.80; | H,4.41; | N,26.17. |

[α]D20=-60.1° (c=1.01%, MeOH)

### Working Example 17

(2R,3R)-3-(2,2-Diethoxyethyl)amino-2-(2,4-difluorophenyl)-1-(1H-1,2,4-triazol-1-yl)-2-butanol (0.25g) was dissolved in dimethylformamide (2ml), and to the solution was added 4-nitrophenyl isocyanate (0.11g). The mixture was stirred for 2 hours at room temperature. The reaction mixture was poured into water (30ml) and extracted with ethyl acetate (30ml). The ethyl acetate layer was washed with water (30ml×2) and saturated aqueous solution of sodium chloride (20ml) successively, and dried over anhydrous magnesium sulfate. The solvent was distilled off under reduced pressure. The residue was purified by silica gel column chromatography (eluent : ethyl acetate/hexane=1/3→1/2) to give 1-(2,2-diethoxyethyl)-1-[(1R,2R)-2-(2,4-difluorophenyl)-2-hydroxy-1-methyl-3-(1H- 1,2,4-triazol-1-yl)propyl]-3-(4-nitrophenyl)urea (0.29g) as a pale yellow powder.
¹H-NMR(CDCl₃)δ : 1.07(1.5H,d,J=7Hz), 1.23(1.5H,d,J=7Hz), 1.30-1.38(6H,m), 3.50-4.10(6.5H,m), 4.36(0.5H,d,J=14Hz), 4.53(0.5H,d,J=14Hz), 4.72-4.77 (0.5H,m), 4.91-5.01(1H,m), 5.26-5.42(2H,m), 6.70-6.84(2H,m), 7.31-7.64(3.5H,m), 7.78(0.5H,s), 7.81(0.5H,s), 8.16-8.23(2.5H,m), 9.02 (0.5H,br), 9.37(0.5H,s).

### Working Example 18

1-(2,2-Diethoxyethyl)-1-[(1R,2R)-2-(2,4-difluorophenyl)-2-hydroxy-1-methyl-3-(1H-1,2,4-triazol-1-yl)propyl]-3-(4-nitrophenyl)urea (0.15g) was dissolved in methanol (3ml), and to the solution was added 1N-hydrochloric acid (2.8ml). The mixture was stirred for 2 hours under reflux. To the reaction mixture was added sodium hydrogencarbonate (0.24g) under ice cooling. To the mixture were added ethyl acetate (40ml) and water (40ml). The ethyl acetate layer was separated, washed with water (30ml) and saturated aqueous solution of sodium chloride (30ml) successively, and dried over anhydrous magnesium sulfate . The solvent was distilled off under reduced pressure to give 1-[(1R,2R)-2-(2,4-difluorophenyl)-2-hydroxy-1-methyl-3-(1H-1,2,4-triazol-1-yl)propyl]-3-(4- nitrophenyl)-2(1H,3H)-imidazolone (0.11g) as a pale yellow powder.
¹H-NMR(CDCl₃)δ : 1.20(3H,d,J=7Hz), 4.19(1H,d,J=14Hz), 5.02(1H,q,J=7Hz), 5.11(1H,d,J=14Hz), 5.41(1H,br), 6.76-6.90(4H,m), 7.41-7.53(1H,m), 7.76 (1H,s), 7.82(1H,s), 7.94(2H,d,J=9.2Hz), 8.34(2H,d,J=9.2Hz).

### Working Example 19

(2R,3R)-3-(2,2-Diethoxyethyl)amino-2-(2,4-difluorophenyl)-1-(1H-1,2,4-triazol-1-yl)-2-butanol (8.9g) was dissolved in dimethylsulfoxide (30ml), and to the solution was added 4-nitrophenyl isocyanate (4.2g). The mixture was stirred for 2 hours at room temperature. The reaction mixture was poured into ice water (200ml) and extracted with ethyl acetate (200ml). The ethyl acetate layer was washed with water (200ml×2) and saturated aqueous solution of sodium chloride (100ml) successively, and dried over anhydrous magnesium sulfate. The solvent was distilled off under reduced pressure. To the residue (13.7g) were added methanol (180ml) and 2N-hydrochloric acid (115ml) successively. The mixture was stirred for 3 hours under reflux. After the mixture was cooled, the resulting crystals were filtered off. To the filtrate was added sodium hydrogencarbonate (19.3g) under ice cooling, and the mixture was concentrated under reduced pressure. To the residue was added ethyl acetate (200ml) and water (200ml). The ethyl acetate layer was separated, washed with water (200ml) and saturated aqueous solution of sodium chloride (100ml) successively, and dried over anhydrous magnesium sulfate. The solvent was distilled off under reduced pressure. The residue was purified by silica gel column chromatography (eluent : ethyl acetate/hexane=2/1) to give 1-[(1R,2R)-2-(2,4-difluorophenyl)-2-hydroxy-1-methyl-3-(1H-1,2,4-triazol-1-yl)propyl]-3-(4-nitrophenyl)-2(1H,3H)-imidazolone (8.6g) as a pale yellow powder. The product was identical with the compound obtained in Working Example 18 in physicochemical properties.

### Working Example 20

1-[(1R,2R)-2-(2,4-Difluorophenyl)-2-hydroxy-1-methyl-3-(1H-1,2,4-triazol-1-yl)propyl]-3-(4-nitrophenyl)-2(1H,3H)-imidazolone (1.0g) was dissolved in acetic acid (15ml), and 10% palladium-carbon (1.0g) was added to the solution. The mixture was stirred at 40 to 45°C for 7 hours under a hydrogen atmosphere. The catalyst was filtered off, and the catalyst was washed with acetic acid (30ml). The filtrate and the washing were combined and concentrated under reduced pressure. The residue was purified by silica gel column chromatography (eluent : ethyl acetate/hexane=9/1→ethyl acetate) and recrystallized from ethyl acetate to give 1-(4-aminophenyl)-3-[(1R,2R)-2-(2,4-difluorophenyl)-2-hydroxy-1-methyl-3-(1H-1,2,4-triazol-1- yl)propyl]-2-imidazolidinone (0.48g).
¹H-NMR(CDCl₃)δ : 1.06(3H,d,J=7Hz), 3.58-3.87(6H,m), 4.46(1H,br), 4.54 (1H,d,J=14Hz), 5.07(1H,d,J=14Hz), 5.80(1H,br), 6.69-6.81(4H,m), 7.31 (2H,d,J=9Hz), 7.35-7.52(1H,m), 7.72(1H,s), 7.92(1H,s).

### Working Example 21

To a mixture of 1-(4-aminophenyl)-3-[(1R,2R)-2-(2,4-difluorophenyl)-2-hydroxy-1-methyl-3-(1H-1,2,4-triazol-1-yl)propyl]-2-imidazolidinone (1.0g) and ethyl orthoformate (1.16ml) were added sodium azide (0.18g) and acetic acid (2ml). The resulting mixture was stirred at room temperature for 20 minutes and further stirred at 80 to 85°C for 1.5 hour under an argon atmosphere. The reaction mixture was cooled, and to the reaction mixture were added water (2ml) and 6N-hydrochloric acid (0.85ml) successively. A 25% aqueous solution of sodium nitrite (0.3ml) was added to the reaction mixture, and the reaction mixture was cooled in an ice bath. The crystals precipitated were collected by filtration, washed with water (20ml), and recrystallized from ethanol to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-imidazolidinone (0.62g) as colorless powdery crystals.
¹H-NMR(CDCl₃)δ : 1.08(3H,d,J=7Hz), 3.70-4.15(4H,m), 4.52(1H,d,J=14Hz), 4.71(1H,q,J=7Hz), 5.12(1H,d,J=14Hz), 5.35(1H,br), 6.74-6.85(2H,m), 7.36-7.49 (1H,m), 7.68(2H,d,J=9Hz), 7.77(1H,s), 7.82(2H,d,J=9Hz), 7.85(1H,s), 8.96(1H,s).

### Working Example 22

To a solution of 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (0.5g) in N,N-dimethylformamide (5ml) was added thionyl chloride (0.09ml), and the mixture was stirred at 50 °C for 30 minutes. The reaction mixture cooled, poured into water (20ml) and extracted with ethyl acetate (20ml×2). The ethyl acetate layer was washed with water (150ml×3) and dried over MgSO4. The solvent was distilled off under reduced pressure. The residue was purified by silica gel column chromatography (eluent : ethyl acetate→ethyl acetate/acetone=1/1) and recrystallized from diisopropyl ether to give 1-(2-chloroethyl)-1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]urea (0.22g) as white powdery crystals.
SIMS (m/z) : 518(MH+)

### Working Example 23

(2R,3R)-2-(2,4-Difluorophenyl)-3-(2-hydroxyethyl)amino-1-(1H-1,2,4-triazol-1-yl)-2-butanol (10.0g) was dissolved in dimethylsulfoxide (50ml), and to the solution was added 4-nitrophenyl isocyanate (5.25g). The mixture was stirred for 1 hour at room temperature. The reaction mixture was poured into ice water (200ml) and extracted with ethyl acetate (200ml). The ethyl acetate layer was washed with water (200ml×2) and saturated aqueous solution of sodium chloride (100ml) successively, and dried over anhydrous magnesium sulfate. The solvent was distilled off under reduced pressure. The residue was recrystallized from ethyl acetate to give 1-[(1R,2R)-2-(2,4-difluorophenyl)-2-hydroxy-1-methyl-3-(1H-1,2,4-triazol-1-yl)propyl]-1-(2-hydroxyethyl)-3-(4-nitrophenyl)urea (5.9g) as yellow powdery crystals.
¹H-NMR(d₆-DMSO)δ : 0.94(1.8H,d,J=7Hz); 1.13(1.2H,d,J=7Hz); 3.40-4.05 (4.4H,m); 4.54,4.58(1H,d,J=14Hz); 4.94,4.97(1H,d,J=14Hz); 5.28(0.6H,q, J=7Hz); 6.08,6.25(2H,br); 6.87-6.96(1H,m); 7.12-7.46(2H,m); 7.59-7.72 (3.4H,m); 8.17-8.30(3H,m): 9.90(0.6H,br).

### Working Example 24

1-[(1R,2R)-2-(2,4-Difluorophenyl)-2-hydroxy-1-methyl-3-(1H-1,2,4-triazol-1-yl)propyl]-1-(2-hydroxyethyl)-3-(4-nitrophenyl)urea (2.6g) was dissolved in dimethylacetamide (40ml), and a solution of thionyl chloride (0.38ml) in ethyl acetate (10ml) was added dropwise to the solution over a period of 30 minutes. After the solution was stirred at room temperature for 20 minutes, a solution of thionyl chloride (0.19ml) in ethyl acetate (5ml) was added dropwise to the solution over a period of 15 minutes. After having been stirred at room temperature for 30 minutes, the mixture was diluted with ethyl acetate (100ml) and washed with water (100ml×2). The ethyl acetate layer was washed with saturated aqueous solution of sodium chloride (50ml) and dried over anhydrous magnesium sulfate. The solvent was distilled off under reduced pressure, and the residue was purified by silica gel column chromatography (eluent : ethyl acetate/hexane=4/1) to give 1-(2-chloroethyl)-1-[(1R,2R)-2-(2,4-difluorophenyl)-2-hydroxy-1-methyl-3-(1H-1,2,4-triazol-1-yl)propyl]-3-(4-nitrophenyl)urea (0.68g) as a pale yellow powder.
¹H-NMR(CDCl₃)δ : 1.09(0.6H,d,J=7Hz); 1.34(2.4H,d,J=7Hz); 3.48-3.60 (1H,m); 3.88-4.28(4H,m); 4.40(0.2H,d,J=14Hz); 4.67(0.8H,d,J=14Hz); 5.13 (0.8H,d,J=14Hz); 5.25(0.2H,d,J=14Hz); 5.28-5.42(1H,br); 6.74-6.91 (3H, m); 7.35-7.72(1.2H,m); 7.62,7.65(2H,d,J=9Hz); 7.79,7.82(1H,s); 7.98 (0.8H, s); 8.23(2H,d,J=9Hz).

### Working Example 25

1-(2-Chloroethyl)-1-[(1R,2R)-2-(2,4-difluorophenyl)-2-hydroxy-1-methyl-3-(1H-1,2,4-triazol-1-yl)propyl]-3-(4- nitrophenyl)urea (0.50g) was dissolved in ethyl acetate (10ml), and a solution of triethylamine (0.28ml) in ethyl acetate (2ml) added dropwise to the solution over a period of 15 minutes. The mixture was stirred at room temperature. At 1 hour and 2 hours afterwards, 0.28 ml each of triethylamine was added, and the mixture was stirred at room temperature for 16 hours. The reaction mixture was diluted with ethyl acetate (40ml), and washed with water (40ml×2). The ethyl acetate layer was washed with 10% aqueous solution of phosphoric acid (40ml), water (40ml) and saturated aqueous solution of sodium chloride (40ml) successively, and dried over anhydrous magnesium sulfate. The solvent was distilled off under reduced pressure. The residue was subjected to silica gel column chromatography (eluent : ethyl acetate/hexane=6/1) to give 1-[(1R,2R)-2-(2,4-difluorophenyl)-2-hydroxy-1-methyl-3-(1H-1,2,4-triazol-1-yl)propyl]-3-(4-nitrophenyl)-2-imidazolidinone (0.21g).
¹H-NMR(CDCl₃)δ : 1.07(3H,d,J=7Hz), 3.70-4.18(4H,m), 4.48(1H,d,J=14Hz), 4.68-4.80(1H,m), 5.11(1H,d,J=14Hz), 5.30(1H,br), 6.73-6.85(2H,m), 7.35-7.48(1H,m), 7.75(2H,d,J=9Hz), 7.77(1H,s) ,7.84(1H,s), 8.24(2H,d, J=9Hz).

### Working Example 26

1-[(1R,2R)-2-(2,4-Difluorophenyl)-2-hydroxy-1-methyl-3-(1H-1,2,4-triazol-1-yl)propyl]-3-(4-nitrophenyl)-2-imidazolidinone (1.0g) was dissolved in a mixture of methanol (36ml) and water (4ml), and to the mixture was added 10% palladium-carbon (0.5g). The mixture was stirred at room temperature for 2 hours under a hydrogen atmosphere. The catalyst was filtered off, and the catalyst was washed with 90% aqueous methanol (60ml). The filtrate and the washing were combined and concentrated under reduced pressure. The residue was purified by silica gel column chromatography (eluent : ethyl acetate→ethyl acetate/acetone=4/1) and recrystallized from ethyl acetate to give 1-[(1R,2R)-2-(2,4-difluorophenyl)-2-hydroxy-1-methyl-3-(1H-1,2,4-triazol-1-yl)propyl]-3-(4-aminophenyl)-2-imidazolidinone (0.45g) as colorless powdery crystals. The product was identical with compound obtained in Working Example 20 in physicochemical properties.

### Working Example 27

1-[(1R,2R)-2-(2,4-Difluorophenyl)-2-hydroxy-1-methyl-3-(1H-1,2,4-triazol-1-yl)propyl]-1-(2-hydroxyethyl)-3-(4-nitrophenyl)urea (2.4g) was dissolved in dimethylacetamide (40ml), and to the solution was added dropwise a solution of thionyl chloride (0.35ml) in ethyl acetate (10ml) over a period of 20 hours. The mixture was stirred at room temperature for 15 minutes, and to the mixture was added dropwise a solution of thionyl chloride (0.17ml) in ethyl acetate (5ml) over a period of 15 minutes. The mixture was stirred at room temperature for 40 minutes, to the mixture was added dropwise a solution of thionyl chloride (0.09ml) in ethyl acetate (2ml) over a period of 10 minutes. The mixture was stirred at room temperature for 40 minutes, and to the mixture was added dropwise triethylamine (2.8ml) at 15 to 18 °C over a period of 5 minutes, and the mixture was stirred at room temperature for 3 hours. The reaction mixture was diluted with ethyl acetate (100ml) and washed with water (100ml×4). The ethyl acetate layer was washed with saturated aqueous solution of sodium chloride (50ml) and dried over anhydrous magnesium sulfate. The solvent was distilled off under reduced pressure. The residue was subjected to silica gel column chromatography (eluent:ethyl acetate/hexane=6/1) to give 1-[(1R,2R)-2-(2,4-difluorophenyl)-2-hydroxy-1-methyl-3-(1H-1,2,4-triazol-1-yl)propyl]-3-(4-nitrophenyl)-2-imidazolidinone (1.2g) as a pale yellow powder. The product was identical with the compound obtained in Working Example 25 in physicochemical properties.

### Working Example 28

To a solution of triphenylphosphine (0.07 g) in tetrahydrofuran (3 ml) was added a 40% solution of azodicarboxylic acid diethyl ester in toluene (0.12 g) at -20 °C, and the mixture was stirred for 10 minutes. To the mixture was added dropwise a solution of 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H- tetrazol-1-yl)phenyl]urea (0.1 g) in tetrahydrofuran (3 ml) over a period of 5 minutes, and the mixture was stirred at room temperature for 2 hours.
The solvent was distilled off under reduced pressure, and the residue was subjected to silica gel flush column chromatography (silica gel : 15 g, eluent ; hexane : ethyl acetate : acetone = 4 : 3 : 3→ethyl acetate). The solid obtained was recrystallized from ethanol to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-imidazolidinone (0.052 g) as white crystals. The product was identical with the compound obtained in Working Example 16 in physicochemical properties.

### Working Example 29

To a solution of 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (2.0g) in N,N-dimethylacetamide-ethyl acetate (1 : 1,20ml) was added dropwise a solution of phosphorus oxychloride (0.51ml) in ethyl acetate (6ml) at an internal temperature of 21 to 23 °C over a period of 1 hour. The mixture was stirred at room temperature for 16 hours. The reaction mixture was diluted with ethyl acetate (30ml) and washed with water (20ml×3). The ethyl acetate layer was dried over anhydrous magnesium sulfate, and the solvent was distilled off under reduced pressure. The residue was recrystallized from ethyl ether (30ml), and the crystals were collected by filtration to give 1-(2-chloroethyl)-1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]urea (1.44g) as white powdery crystals.
¹H-NMR(d₆-DMSO)δ : 1.02,1.16(3H,d,J=7Hz); 3.61-4.09(4.5H,m); 4.43, 4.57 (1H,d, J=14Hz); 4.87,4.97(1H,d,J=14Hz); 5.20(0.5H,q,J=7Hz): 5.98(1H, br); 6.89-6.98(1H,m); 7.13-7.48(2H,m); 7.64-7.85(5H,m); 8.31(1H,s); 9.02, 9.22 (1H,s); 10.02(1H,s)
SIMS (m/z) : 500(MH+)

### Working Example 30

To a solution of 1-(2-chloroethyl)-1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]urea (1.2g) in N,N-dimethylacetamide-ethyl acetate (3 : 5,16ml) was added dropwise a solution of triethylamine (0.96ml) in ethyl acetate (2ml) at an internal temperature of 0 to 2°C over a period of 15 minutes. After the mixture was stirred at room temperature for 3 hours, ice water (20ml) was added to the mixture. The mixture was extracted with ethyl acetate (20ml×2). The extract was washed with water (20ml), 5% aqueous solution of phosphoric acid (20ml×2), saturated aqueous solution of sodium hydrogencarbonate (20ml) and saturated aqueous solution of sodium chloride (20ml×2) successively. The ethyl acetate layer was dried over anhydrous magnesium sulfate, and the solvent was distilled off under reduced pressure. The residue was crystallized from ethyl acetate (10ml), and the crystals were collected by filtration. The crystals were recrystallized from ethanol (40ml) and dried over phosphorus pentoxide at 40°C for 1 hour to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-imidazolidinone (0.765g) as white crystals. The product was identical with the compound obtained in Working Example 16 in physicochemical properties.

### Working Example 31

To a solution of 1-[(1R,2R)-2-(2,4-difluorophenyl)-2-hydroxy-1-methyl-3-(1H-1,2,4-triazol-1-yl)propyl]-1-(2-hydroxyethyl)-3-(4-nitrophenyl)urea (3.0g) in N,N-dimethylacetamide-ethyl acetate (1 : 1,30ml) was added dropwise a solution of phosphorus oxychloride (0.88ml) in ethyl acetate (10ml) at 21 to 22°C over a period of 20 minutes. After the mixture was stirred at room temperature for 16 hours, triethylamine (8.8ml) was added dropwise to the mixture at -5 °C over a period of 20 minutes. The mixture was stirred at room temperature for 2 hours. The reaction mixture was diluted with ethyl acetate (100ml) and washed with water (100ml×2). The ethyl acetate layer was washed with 5% aqueous solution of phosphoric acid (100ml×2), saturated aqueous solution of sodium bicarbonate (100ml) and saturated aqueous solution of sodium chloride (100ml) successively, and dried over anhydrous magnesium sulfate. The solvent was distilled off under reduced pressure. The residue was subjected to silica gel column chromatography (eluent : ethyl acetate/hexane=6/1) to give 1-[(1R,2R)-2-(2,4-difluorophenyl)-2-hydroxy-1-methyl-3-(1H-1,2,4-triazol-1-yl)propyl]-3-(4-nitrophenyl)-2-imidazolidinone (2.3g) as a pale yellow powder. The product was identical with the compound obtained in Working Example 25 in physicochemical properties.

### Working Example 32

1-[(1R,2R)-2-(2,4-Difluorophenyl)-2-hydroxy-1-methyl-3-(1H-1,2,4-triazol-1-yl)propyl]-3-(4-nitrophenyl)-2-imidazolidinone (1.0 g) was dissolved in a mixture of ethyl acetate (18ml) and water(2ml). To the mixture was added 10% palladium-carbon (0.5g), and the mixture was stirred at room temperature under a hydrogen atmosphere for 3 hours. The catalyst was filtered off and the catalyst was washed with ethyl acetate (50ml). The filtrate and the washing were combined and concentrated under reduced pressure. The residue was crystallized from ethyl acetate to give 1-(4-aminophenyl)-3-[(1R,2R)-2-(2,4-difluorophenyl)-2-hydroxy-1-methyl-3-(1H- 1,2,4-triazol-1-yl)propyl-2-imidazolidinone (0.35 g ) as colorless powdery crystals. The product was identical with the compound obtained in Working Example 20 in physicochemical properties. The mother liquor was concentrated under reduced pressure. The residue was purified by silica gel column chromatography (eluent : ethyl acetate*/*acetone=3*/*1) and crystallized from a mixture of ethyl acetate and diisopropylether to give additional 1-(4-aminophenyl)-3-[(1R,2R)-2-(2,4-difluorophenyl)-2-hydroxy-1-methyl-3-(1H-1,2,4-triazol-1-yl)propyl]-2-imidazolidinone (0.35 g ) as colorless powdery crystals.

### Working Example 33

A mixture of (2R,3S)-2-(2,4-difluorophenyl)-3-methyl-2-(1H-1,2,4-triazol-1-yl)methyloxirane (20 g ) and 2-(N-benzyl)aminoethanol (80g) was heated at 160°C under a nitrogen atmosphere for 8 hours. After the reaction mixture had been cooled, ethyl acetate (200ml) was added to the reaction mixture. The mixture was extracted with 4N hydrochloric acid (205ml). The hydrochloric acid layer was separated and neutralized (pH7.0±0.2) by adding 2N sodium hydroxide (410ml) under ice cooling. The mixture was extracted with ethyl acetate (150ml ×2), and the extract was washed with saturated aqueous solution of sodium chloride (100ml) and water (100ml×2), successively. The ethyl acetate layer was dried over anhydrous magnesium sulfate and concentrated under reduced pressure. To the residue was added diisopropyl ether (50ml), and the mixture was stirred at room temperature for 20 hours and then under ice cooling for 3 hours. The crystals were collected by filtration, washed with cold diisopropyl ether (5ml) and dried at 40°C under reduced pressure to give (2R,3R)-3-[N-benzyl-N-(2-hydroxyethyl)amino]-2-(2,4-difluorophenyl)-1-(1H-1,2,4-triazol-1-yl)-2-butanol (17.6g) as white powdery crystals.
Yield 55%
Melting point 105-107°C

| Elemental Analysis: C₂₁H₂₄F₂N₄O₂ | | | |
|---|---|---|---|
| Calcd.(%) : | C,62.67; | H,6.01; | N,13.92 |
| Found (%) : | C,62.45; | H,6.07; | N,13.90 |

### Working Example 34

A mixture of (2R,3S)-2-(2,4-difluorophenyl)-3-methyl-2-(1H-1,2,4-triazol-1-yl)methyloxirane (20 g ) and 2-(N-benzyl)aminoethanol (80 g ) was heated under a nitrogen atmosphere at 160°C for 8 hours. After having been cooled, the reaction mixture was dissolved in ethyl acetate (200ml). The solution was extracted with 4N hydrochloric acid (205ml). The hydrochloric acid layer was separated, neutralized with 2N sodium hydroxide (410ml) (pH7.0±2) under ice cooling and extracted with ethyl acetate (150ml×2). The extract was washed with saturated aqueous solution of sodium chloride (100ml) and water (100ml×2), successively to give a solution of (2R,3R)-3-[N-benzyl-N-(2-hydroxyethyl)amino]-2-(2,4-difluorophenyl)-1-(1H-1,2,4-triazol-1-yl)-2-butanol in ethyl acetate.
To the solution was added 5% palladium-carbon (6.3g). To the resulting mixture was added dropwise formic acid (14.3 g) over a period of 30 minutes with stirring. After the addition was complete, the mixture was stirred at 40°C for 2 hours. The reaction mixture was cooled, and the catalyst was filtered off. The catalyst was washed with ethyl acetate (120ml). The filtrate and the washing were combined, and anhydrous magnesium sulfate was added to the combined solution. The mixture was stirred for 30 minutes. The insoluble substance was filtered off and the insoluble substance was washed with ethyl acetate (50ml). The filtrate and the washing were combined to give a solution of (2R,3R)-2-(2,4-difluorophenyl)-3-(2-hydroxyethyl)amino-1-(1H-1,2,4-triazol-1-yl)-2-butanol in ethyl acetate.
To the solution were added triethylamine (7.63g) and phenyl 4-(1H-tetrazol-1-yl)phenylcarbamate (21.6g), and the mixture was stirred at 50°C for 8 hours. The reaction mixture was further stirred at room temperature for 1 hour and under ice cooling for 1 hour. The resulting crystals were collected by filtration, washed with cold ethyl acetate (20ml) and dried at 45°C under reduced pressure to give 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (34.5g)as white powdery crystals. The product was identical with the compound obtained in Working Example 13 in physicochemical properties.

### Working Example 35

1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea (20g) was dissolved in a mixture of 1-methyl-2-pyrrolidone (120ml) and ethyl acetate (200ml). To the solution was added dropwise phosphorus oxychloride (5g) in ethyl acetate (80ml) over a period of 30 minutes. The internal temperature was kept at 50°C all through the addition After the addition was complete, the mixture was stirred at 50°C for 30 minutes. The reaction mixture was cooled to 5°C, and piperidine (23.9g) was added over a period of 2 minutes. The reaction mixture was stirred at 5°C for 30 minutes. To the mixture was added water (150ml), and the mixture was stirred for 30 minutes. The pH of the reaction mixture was adjusted to 6.5 by adding 1N-hydrochloric acid. The ethyl acetate layer was separated and the aqueous layer was extracted with ethyl acetate (100ml). The ethyl acetate layers were combined, washed with water (100ml×2) and concentrated under reduced pressure. To the residue was added 50% aqueous solution of ethanol (240ml), and the mixture was stirred at room temperature for 8 hours. The resulting crystals were collected by filtration, washed with cold ethyl alcohol (28ml) and dried under reduced pressure to give 1-[(1R,2R)-2-(2,4-difluorophenyl)-2-hydroxy-1-methyl-3-(1H-1,2,4-triazol-1-yl)propyl]-3-[4-(1H-tetrazol-1-yl)phenyl]-2-imidazolidinone (16g) as white crystals. The product was identical with the compound obtained in Working Example 16 in physicochemical properties.

## Claims

1. A compound of formula: 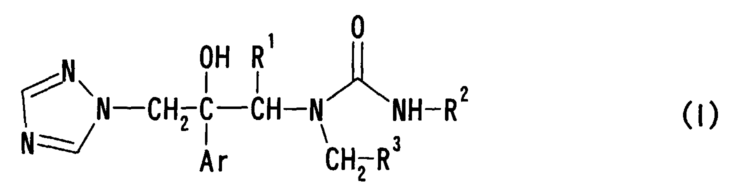 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; R³ is a group of the formula -CH₂Y (wherein Y is a hydroxyl group or a halogen atom) or a formyl group which may be protected, or a salt thereof.

2. A compound of formula: 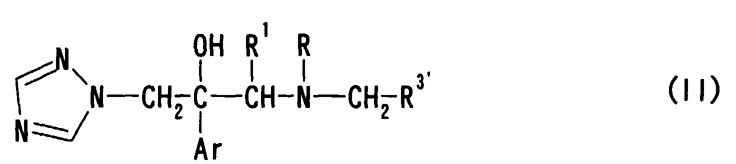 wherein Ar is a phenyl group which may be substituted; R is a hydrogen atom or a benzyl group which may be substituted; R¹ is a hydrogen atom or a lower alkyl group; R^{3'} is a hydroxymethyl group or a protected formyl group, or a salt thereof.

3. A compound as claimed in Claim 1 or 2, wherein R¹ is methyl.

4. A compound as claimed in Claim 1 or 2, wherein Ar is a phenyl group substituted by 1 or 2 halogen atoms.

5. A compound as claimed in Claim 1 or 2, wherein Ar is 2,4-difluorophenyl or 2-fluorophenyl.

6. A compound as claimed in Claim 1, wherein R² is a substituted phenyl group.

7. A compound as claimed in Claim 1, wherein R² is a phenyl group substituted by a halo(C₁₋₆)alkoxy group.

8. A compound as claimed in Claim 1, wherein R² is 4-(2,2,3,3-tetrafluoropropoxy)phenyl or 4-(1,1,2,2-tetrafluoroethoxy)phenyl.

9. A compound as claimed in Claim 1, wherein R² is a phenyl group substituted by a 5-membered aromatic heterocyclic group.

10. A compound as claimed in Claim 1, wherein R² is a phenyl group substituted by pyrazolyl, imidazolyl, 1,2,3-triazolyl, 1,2,4-triazolyl, or tetrazolyl.

11. A compound as claimed in Claim 1, wherein R² is a phenyl group substituted by nitro.

12. A compound as claimed in Claim 1 or 2, wherein R³ or R^{3'} is a group of the formula 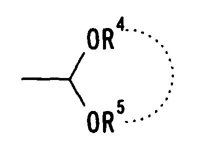 wherein R⁴ and R⁵ may be the same or different and each is an aliphatic hydrocarbon group or R⁴ and R⁵ taken together are an alkylene group of 2 or 3 carbon atoms.

13. A compound as claimed in Claim 12, wherein R⁴ and R⁵ each is a methyl group or an ethyl group.

14. A compound as claimed in Claim 2, wherein R is a benzyl group.

15. A compound as claimed in Claim 2, wherein R is a hydrogen atom.

16. A compound as claimed in Claim 2, which is (2R,3R)-2-(2,4-difluorophenyl)-3-(2-hydroxyethyl)amino-1-(1H-1,2,4-triazol-1-yl)-2-butanol.

17. A compound as claimed in Claim 2, which is (2R,3R)-3-[N-benzyl-N-(2-hydroxyethyl)amino]-2-(2,4-difluorophenyl)-1-(1H-1,2,4-triazol-1-yl)-2-butanol.

18. A compound as claimed in Claim 1, which is 1-[(1R,2R)-2-(2,4-difluorophenyl)-2-hydroxy-1-methyl-3-(1H-1,2,4-triazol-1-yl)propyl]-1-(2-hydroxyethyl)-3-[4-(1H-tetrazol-1-yl)phenyl]urea.

19. A process for producing a compound of formula: 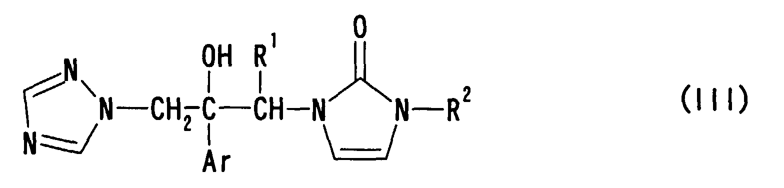 wherein the respective symbols have the same meanings as defined below, or a salt thereof, which comprises subjecting a compound of formula: 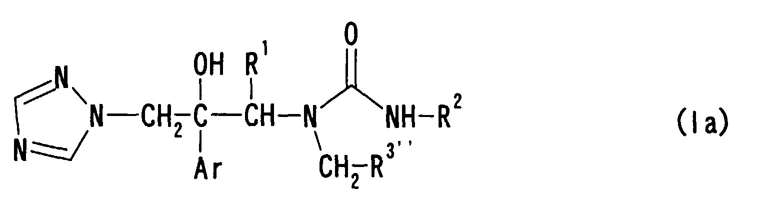 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; R^{3''} is a formyl group which may be protected, or a salt thereof, to a cyclization reaction.

20. A process as claimed in Claim 19, wherein the cyclization reaction is conducted in the presence of an acid.

21. A process as claimed in Claim 20, wherein the acid is an inorganic acid.

22. A process for producing a compound of formula: 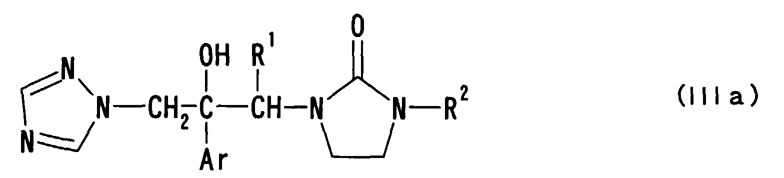 wherein the respective symbols have the same meanings as defined below, or a salt thereof, which comprises subjecting a compound of formula: 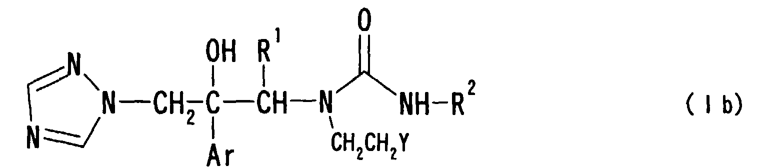 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted; Y is a hydroxyl group or a halogen atom, or a salt thereof, to a cyclization reaction.

23. A process as claimed in Claim 22, wherein the compound in which Y is a hydroxyl group is subjected to a cyclization reaction in the presence of a halogenating agent and a base.

24. A process as claimed in Claim 23, wherein the halogenating agent is a thionyl halide or a halogenated phosphorus compound.

25. A process as claimed in Claim 22, wherein the compound in which Y is a halogen atom is subjected to a cyclization reaction in the presence of a base.

26. A process as claimed in Claim 23 or 25, wherein the base is an organic base.

27. A process for producing a compound of formula: 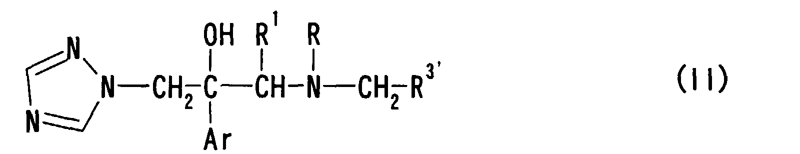 wherein the respective symbols have the same meanings as defined below, or a salt thereof, which comprises reacting a compound of formula: 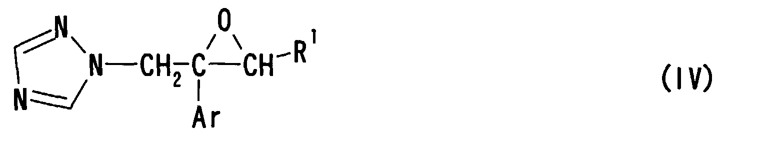 wherein Ar is a phenyl group which may be substituted; R¹ is a hydrogen atom or a lower alkyl group, or a salt thereof, with a compound of formula: 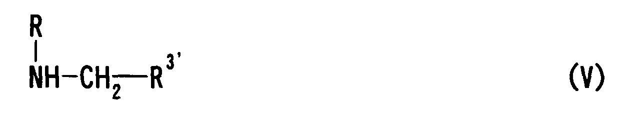 wherein R is a hydrogen atom or a benzyl group which may be substituted, R^{3'} is a hydroxymethyl group or a protected formyl group.

28. A process for producing a compound of formula: 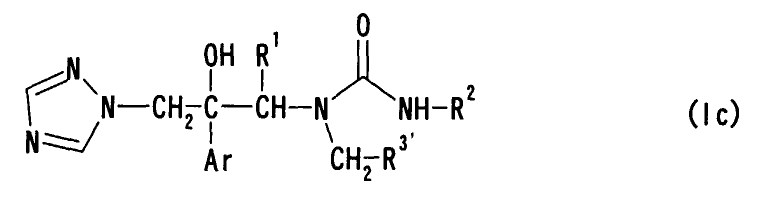 wherein the respective symbols have the same meanings as defined below, or a salt thereof, which comprises reacting a compound of formula: 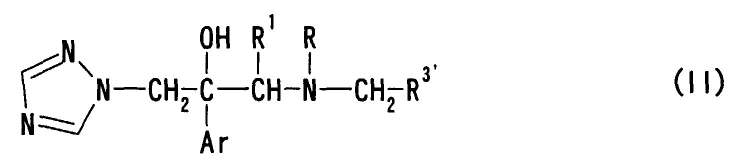 wherein Ar is a phenyl group which may be substituted; R is a hydrogen atom or a benzyl group which may be substituted; R¹ is a hydrogen atom or a lower alkyl group; R^{3'} is a hydroxyethyl group or a protected formyl group, or a salt thereof, with a compound of formula: 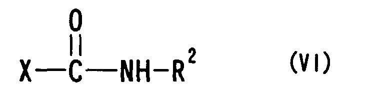 wherein R² is an aliphatic or aromatic hydrocarbon group which may be substituted or an aromatic heterocyclic group which may be substituted; X is a leaving group, or with a compound of formula:
OCN―R² (VII)
wherein R² has the same meaning as defined above, if necessary, after the compound of formula (II) or a salt thereof is subjected to a debenzylation reaction.

29. A process for producing a compound of formula: 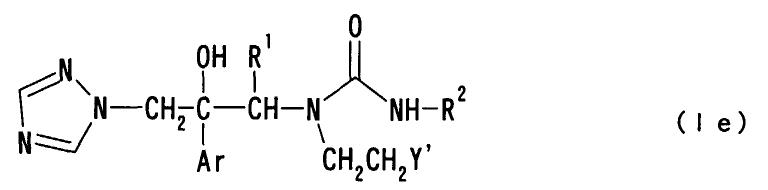 wherein Y' is a halogen atom; the other symbols have the same meanings as below, or a salt thereof, which comprises reacting a compound of formula: 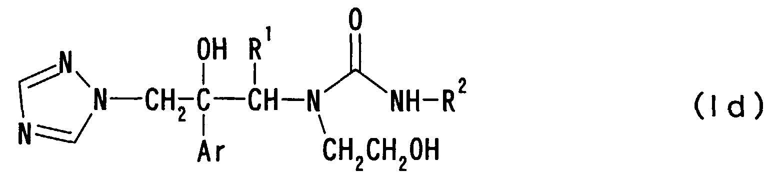 wherein Ar is a phenyl group which may be substituted; R¹ is a hydrogen atom or a lower alkyl group; R² is an aliphatic or aromatic hydrocarbon group which may be substituted or an aromatic heterocyclic group which may be substituted, or a salt thereof, with a halogenating agent.

30. A process for producing a compound of formula: 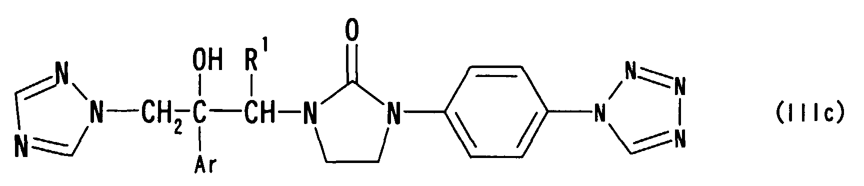 wherein the respective symbols have the same meanings as defined below, or a salt thereof, which comprises reacting a compound of formula: 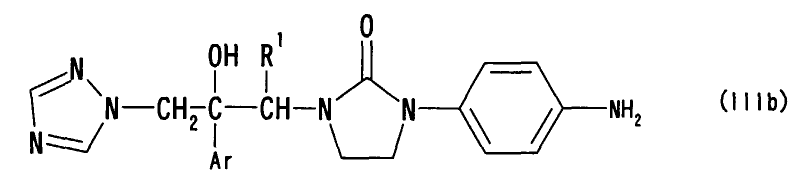 wherein Ar is a phenyl group which may be substituted; R¹ is a hydrogen atom or a lower alkyl group, or a salt thereof, with a lower alkyl ester of orthoformic acid and an alkali metal azide.
